(19) Europäisches Patentamt
European Patent Office
Office européen des brevets

(11) **EP 2 980 223 A2**

(12) **EUROPEAN PATENT APPLICATION**

(43) Date of publication:
03.02.2016 Bulletin 2016/05

(51) Int Cl.:
*C12Q 1/68* (2006.01)   *H01J 37/20* (2006.01)
*G01N 33/58* (2006.01)

(21) Application number: 15178875.9

(22) Date of filing: 29.07.2015

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: 31.07.2014 US 201414449088

(71) Applicant: **FEI Company**
**Hillsboro, OR 97124-5793 (US)**

(72) Inventors:
• **Utlaut, Mark**
**Scappoose, OR 97056 (US)**
• **Parker, N. William**
**Hillsboro, OR 97124 (US)**

(74) Representative: **Bakker, Hendrik**
**FEI Company**
**Patent Department**
**P.O. Box 1745**
**5602 BS Eindhoven (NL)**

(54) **FUNCTIONALIZED GRIDS FOR LOCATING AND IMAGING BIOLOGICAL SPECIMENS AND METHOD OF USING THE SAME**

(57)    A functionalized specimen support for use in charged particle microscopy is provided that includes a specimen support surface configured to support specimens (e.g. DNA strands 1051) during an interrogation of the specimens with a charged particle microscope, the specimen support surface having functionalized sites 1031, each functionalized site configured to maintain position of a portion of one of the specimens at the functionalized site by way of attachment, attraction, or a combination thereof.

FIG. 10A

EP 2 980 223 A2

FIG. 10B

## Description

### Technical Field

The following is directed to methods, apparatuses, and systems for locating a specimen on a relatively large specimen support in applications involving charged particle apparatuses. Various embodiments of the disclosure more specifically relate to methods of locating DNA on a sample support in DNA sequencing applications involving an STEM.

### Background

Over the last century, the development of charged particle microscopes (CPMs) has led to the observation of natural phenomena at magnifications far greater than can be achieved in optical microscopy. One such CPM is a scanning transmission electron microscope (STEM). In a STEM, a focused beam of high-energy electrons is scanned across a thin sample. Electrons in the beam interact with the sample as they pass through it and are collected below the sample. Different imaging and analysis techniques use different characteristics of the transmitted electrons to form an image or to determine properties of the sample. STEM imaging involves obtaining information about the sample from the number of the electrons impacting a detector as a focused electron beam is scanned along the sample surface. A STEM detector used in STEM imaging may be a scintillator-photomultiplier detector, known as an Everhart-Thornley detector, a PIN solid state detector, or any other suitable detector. A STEM detector is typically sufficiently fast to allow data collection as the primary electron beam scans a region of the sample surface. A typical scan, for example rastering the beam over a thousand rows with a thousand scan points in each row, may take about one second and can generate over a million pixels of information, and so the detector preferably can provide readings at a rate of at least one million pixels per second. A typical high accuracy STEM imaging circuit can obtain a reading in microseconds, preferably less than 100 $\mu$s, less than 50 $\mu$s, and more preferably less than 10 $\mu$s.

In recent years, there has been increasing interest in adapting STEM technologies for high-throughput applications. Of particular interest is the use of STEMs for high-throughput DNA sequencing. Now in the third generation of development, DNA sequencing technologies aim to provide systems that deliver single molecule data and very long read capabilities (*i.e.,* reading DNA strands of 20,000 or more base pairs) at shorter sequencing times and higher throughput capacities.

One factor affecting the efficiency of STEM-based DNA sequencing, and the analysis by electron microscope of very small (*e.g.,* nano-scale) specimens in general, is the time it takes to locate the specimens to be analyzed. A typical DNA specimen is a DNA strand having a diameter of about 2 nanometers (nm) and a length ranging up to approximately 12 microns ($\mu$m) in length (corresponding to -30,000 bases), which corresponds to a lengthwise cross-sectional area of less than about 24,000 $nm^2$. By comparison, in conventional STEM analysis, samples are typically presented for analysis on a thin carbon film supported by a 3 mm diameter microscope grid, which corresponds to a sample support area of 7.1 x $10^{12}$ $nm^2$. Thus, a typical DNA specimen will at most occupy a fractional area of a 3 mm diameter grid corresponding to 3.4 x $10^{-9}$, and any analysis of a DNA specimen by an STEM would thus involve first locating the specimen over an area nine orders of magnitude greater than the specimen.

To illustrate the significance of the ratio between DNA specimen size and the specimen support area on specimen processing times, consider the length of time it would take to scan an entire area of a 3 mm diameter grid support using a STEM beam at a resolution of 2 nm, which is about the diameter of a DNA strand and representative of the minimum resolution required to detect the presence of DNA. Assuming a conventional high-speed imaging rate of 40 MHz, it would take approximately 12.3 hours of imaging time to acquire all 1.8 x $10^{12}$ pixels of the sample grid. Although 12.3 hours represents a maximum time for locating a DNA specimen, the example demonstrates the relevance of sample location time to realizing higher throughput in STEM-based DNA sequencing and other applications involving small biological specimens.

Thus, there is a need for faster methods of locating nanoscale biological specimens on sample grids in charged particle microscopy applications such as, for example, STEM-based DNA sequencing. There is also a need for systems and apparatuses facilitating faster location of nanoscale biological specimens in charged particle microscopy applications such as, for example, STEM-based DNA sequencing.

### Summary

In some embodiments of the disclosure, a functionalized specimen support for use in charged particle microscopy is provided that includes a specimen support surface configured to support a specimen during an interrogation of the specimen with a charged particle microscope, the specimen support surface having a functionalized site configured to maintain a position of the specimen at the functionalized site by way of attachment, attraction, or a combination thereof. In some embodiments, the specimen includes a strand of DNA. In some embodiments, the specimen includes a strand of DNA labelled with a light-emitting label and/or heavy atom tags.

In some embodiments of the disclosure, a specimen preparation for an electron microscope is provided that includes: a specimen support, wherein a surface of the specimen support includes a plurality of sites, each site being functionalized to maintain a position of a specimen at the site by way of a localized attractive force, an attachment mechanism, or a combination thereof; and a plurality of specimens, each specimen adhered to one of the sites. In some embodiments, the plurality of specimens comprises strands of DNA. In some embodiments, the plurality of specimens includes strands of DNA labelled with a light-emitting label and/or heavy atom tags.

In some embodiments of the disclosure, a method of locating specimens on a specimen support is provided that includes locally scanning a plurality of pre-defined locations on a specimen support for a presence of a specimen using a charged particle beam, wherein a position of a portion of the specimen is fixed at one of the pre-defined locations. In some embodiments, the specimen includes a DNA strand and the charged particle beam is an electron beam of a STEM having a resolution greater than or equal to a minimum resolution required to determine a position of a portion of the DNA strand and less than a minimum resolution required to sequence the DNA strand.

In some embodiments of the disclosure, a method of locating specimens on a specimen support is provided that includes: loading a microscope with a specimen support having a surface having a plurality of pre-defined attachment locations, wherein a specimen having a light-emitting label is attached to one of the pre-defined attachment locations; capturing local images of areas of the surface, each local image encompassing one of the pre-defined attachment locations and captured at a resolution sufficient to detect light from the light-emitting label; and determining which of the pre-defined attachment locations is occupied by the specimen by evaluating the local images for a presence of a light signature of the light-emitting label.

In some embodiments of the disclosure, a method of locating specimens on a specimen support is provided that includes: capturing an image of a surface of a specimen support populated with labeled specimens, wherein a resolution of the image is sufficient to determine an approximate location of a label of one of the labeled specimens from the image; and processing the image to determine an approximate position of the label.

In some embodiments of the disclosure, a non-transitory machine-readable storage medium is provided that contains executable instructions that, when executed, cause one or more processors to direct a charged particle beam to locally scan a plurality of pre-defined locations on a surface of a specimen support for a presence of a specimen using a charged particle beam, wherein the specimen is attached to one of the pre-defined locations. In some embodiments, the specimen includes a DNA strand and the charged particle beam is an electron beam of a STEM having a resolution greater than or equal to a minimum resolution required to determine a position of a portion of the DNA strand and less than a minimum resolution required to sequence the DNA strand.

In some embodiments of the disclosure, a scanning transmission electron microscope system for high-throughput sample processing is provided that includes a scanning transmission electron microscope and a specimen support, wherein a surface of the specimen support includes a plurality of locations, each location having an attachment site functionalized to fix a position of a portion of a specimen to the surface at the location.

The foregoing has outlined rather broadly the features and technical advantages of the present invention in order that the detailed description of the invention that follows may be better understood. Additional features and advantages of the invention will be described hereinafter. It should be appreciated by those skilled in the art that the conception and specific embodiments disclosed may be readily utilized as a basis for modifying or designing other structures for carrying out the same purposes of the present invention. It should also be realized by those skilled in the art that such equivalent constructions do not depart from the invention as set forth in the appended claims.

**Brief Description Of The Drawings**

For a more thorough understanding of the present disclosure, and advantages thereof, reference is now made to the following descriptions taken in conjunction with the accompanying drawings, in which:

FIG. 1 is a flow diagram of a method of locating and analyzing specimens on a specimen support according to an embodiment of the disclosure.

FIG. 2 is a flow diagram of a method of locating and sequencing DNA on a specimen support according to an embodiment of the disclosure.

FIG. 3 is a flow diagram of a method of locating and analyzing specimens according to an embodiment of the disclosure.

FIG. 4 is a flow diagram of a method of locating and sequencing DNA on a specimen support according to an embodiment of the disclosure.

FIG. 5 is a flow diagram of a method of locating and analyzing specimens according to an embodiment of the disclosure.

FIG. 6 is a flow diagram of a method of locating and sequencing DNA on a specimen support according to an embodiment of the disclosure.

FIG. 7 is an illustrative diagram of a strategy for sequencing a long-read DNA strand with a STEM according to an embodiment of the disclosure.

FIG. 8 is an illustration showing how signal strength is expected to vary along a fast scan axis of a line scan generated by a STEM centered on the axis of the long-read DNA strand of FIG. 7.

FIG. 9 is an illustration showing how signal strength is expected to vary along a fast scan axis of a line scan generated by a STEM offset from the axis of the long-read DNA strand of FIG. 7.

FIG. 10A is a simplified schematic illustration of a functionalized specimen support in accordance with an embodiment of the disclosure.

FIG. 10B is a simplified schematic diagram of a close-up of a single square grid of a functionalized specimen support in accordance with an embodiment of the disclosure.

FIG. 10C is a simplified schematic diagram of a close-up of a single square grid of a functionalized specimen support in accordance with an embodiment of the disclosure.

FIG. 10D is a simplified schematic diagram of a close-up of a single square grid of a functionalized specimen support in accordance with an embodiment of the disclosure.

FIG. 11A is a simplified schematic illustration of a functionalized specimen support in accordance with an embodiment of the disclosure.

FIG. 11B is a simplified schematic diagram of a close-up of a single rectangle grid of a functionalized specimen support in accordance with an embodiment of the disclosure.

FIG. 11C is a simplified schematic diagram of a close-up of a single rectangle grid of a functionalized specimen support in accordance with an embodiment of the disclosure.

FIG. 11D is a simplified schematic diagram of a close-up of a single rectangle grid of a functionalized specimen support in accordance with an embodiment of the disclosure.

FIG. 12A is a simplified schematic illustration of a functionalized specimen support in accordance with an embodiment of the disclosure.

FIG. 12B is a simplified schematic diagram of a close-up of a single rectangle grid of a functionalized specimen support in accordance with an embodiment of the disclosure.

FIG. 12C is a simplified schematic diagram of a close-up of a single rectangle grid of a functionalized specimen support in accordance with an embodiment of the disclosure.

FIGS. 13A and B are exemplary electron microscope grids that may be adapted in accordance with embodiments of the disclosure.

FIG. 14A is an image of a 3 mm diameter Gilder Reference Locater Grid that may be adapted in accordance with embodiments of the disclosure

FIG. 14B and 14C shows another type of finder grid that may be adapted in accordance with embodiments of the disclosure.

FIG. 15 is a block diagram of a scanning transmission electron microscope system according to an embodiment of the disclosure.

FIG. 16 is a scanning transmission electron microscope in accordance with an embodiment of the disclosure.

## Detailed Description

In the drawings and description that follow, like parts are typically marked throughout the specification and drawings with the same reference numerals, respectively. In addition, similar reference numerals may refer to similar components in different embodiments disclosed herein. The drawing figures are not necessarily to scale. Certain features of the invention may be shown exaggerated in scale or in somewhat schematic form and some details of conventional elements may not be shown in the interest of clarity and conciseness. The present invention is susceptible to embodiments of different forms. Specific embodiments are described in detail and are shown in the drawings, with the understanding that the present disclosure is not intended to limit the invention to the embodiments illustrated and described herein. It is to be fully recognized that the different teachings of the embodiments discussed herein may be employed separately or in any suitable combination to produce desired results.

In the following discussion and in the claims, the terms "including" and "comprising" are used in an open-ended fashion, and thus should be interpreted to mean "including, but not limited to ...." To the extent that any term is not specially defined in this specification, the intent is that the term is to be given its plain and ordinary meaning. Furthermore, use of the term "and/or" herein shall be construed as an "inclusive" or, and not an "exclusive" or. For example, used herein the phrase "A and/or B" would mean "A, B, or A and B." As another example, used herein the phrase "A, B, and/or C" would mean "A, B, C, or any combination thereof." Further, whenever the terms "automatic," "automated," or similar terms are used herein, those terms will be understood to include manual initiation of the automatic or automated process or step. Still further, the term "specimens" will be used herein to convey both singular and plural meanings, except where such use would conflict with the context and in cases where the singular case is separately addressed by the disclosure. For example, an embodiment wherein *"specimens are scanned with a beam"* is intended to convey an embodiment wherein *"a specimen is scanned with a beam"* as well as the embodiment wherein *"specimens are scanned with a beam."*

Disclosed herein are methods for locating specimens on a relatively large surface of a specimen support and then imaging the specimens with a CPM such as a STEM. Various embodiments of the methods include isolating positions of specimens at different pre-defined locations on the surface of the specimen support, the cumulative area of the pre-defined locations being less than a total area of the surface. Embodiments, such as the embodiments of methods 100, 200, 300, 400, 500, and 600 disclosed herein, may advantageously reduce the amount of time required for locating a sample. The time savings that may be achieved by methods 200, 400, and 600 may be particularly advantageous in third generation DNA sequencing, including STEM-based high-throughput DNA sequencing, where the importance of sequencing time predominates over the time required to preparing specimens for sequencing.

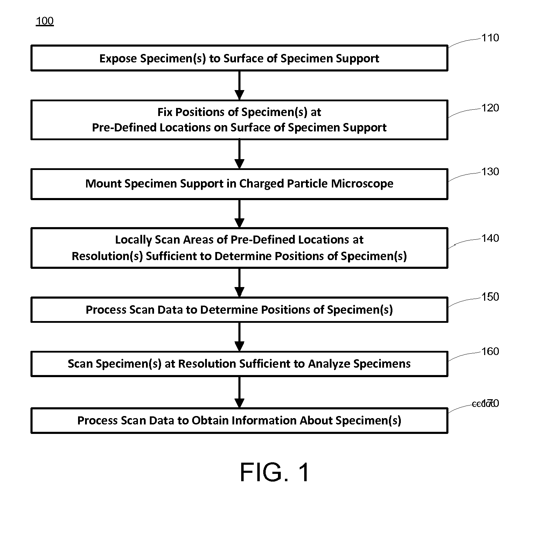

In some embodiments, the method includes locally scanning the pre-defined locations at resolutions sufficient to determine positions of the specimens and then scanning located specimens again at a higher resolution to analyze the specimens. Referring to FIG. 1, a flow diagram of a method 100 of locating and imaging specimens on a sample support in accordance with an embodiment of the disclosure is shown. Method 100 may begin by preparing specimens on a surface of a specimen support. Preparation may begin at Block 110 by exposing the specimens to a surface of a specimen support comprising a plurality of pre-defined locations and functionalized to fix positions of the specimens at the pre-defined locations. The sizes (surface areas) of the pre-defined locations may be less than or equal to approximately $(20 \ \mu m)^2$, alternatively less than or equal to approximately $(10 \ \mu m)^2$, alternatively less than or equal to approximately $(5 \ \mu m)^2$. In various embodiments, exposing the specimens to the surface of the specimen support includes bringing the specimens in proximity to the attachment sites to facilitate fixing of the specimens by the attachment site.

A "pre-defined location," as used herein, refers to a location on a surface of a specimen support that is known prior to use of the specimen support for locating specimens. For example, coordinates for pre-defined locations of a surface of the specimen support may be stored in a separate machine-readable memory, which may be accessed to carry out one or more of the localized scanning steps described herein. A "pre-defined location" is also used herein to refer to a location on a surface of a specimen support that is readily ascertainable by the presence of location-marking features of the specimen support.

In various embodiments, functionalization of the support surface takes the form of attachment sites located at or proximate to the pre-defined locations and functionalized to fix a position of the specimens at the pre-defined locations. As used herein, an "occupied pre-defined location" refers to a pre-defined location at which a position of a specimen has been fixed. In various embodiments, the attachment sites are configured such that each pre-defined location can only be occupied by a single specimen.

Preparation of the specimens may continue to Block 120 by fixing positions the specimens at the pre-defined locations. Except where indicated otherwise, "fixing a position of a specimen" and "maintaining a position of a specimen," as used herein, refer to any one of fixing a position of a portion of the specimen (*e.g.,* a DNA strand attached to a surface at one end and free at the other), fixing a position and an orientation of the specimen (*e.g.,* a DNA strand attached to a surface at one end and oriented lengthwise along a fixed radial direction extending from the position of the attached end), eliminating all translational and orientational degrees of freedom of the sample relative to the location and/or site (*e.g.,* no part of the DNA strand moves relative to the surface).

In various embodiments, fixing the positions of the specimens at the pre-defined locations comprises fixing a position of each specimen at a different pre-defined location. In some embodiments, the pre-defined locations comprise attachment sites functionalized to fix positions of the specimens at the pre-defined locations. Each attachment site may be functionalized to fix the position of a specimen according to one or more mechanisms. Suitable mechanisms include, but are not limited to, chemical attachment (*e.g.,* a bond-forming chemical reaction between the attachment site and the specimen), exertion of an attractive force (*e.g.,* exertion of an electrostatic field opposite in sign to an electrostatic charge of the specimen; intermolecular forces between molecules of the attachment site and the specimen), adhesion, and mechanical engagement (*e.g.,* via a nanoscale interlocking mechanism). In various embodiments, the mechanisms are executed by bringing the specimens with a proximity to the attachment sites and creating any additional conditions necessary to trigger the specific mechanisms employed by the attachment sites. Attachment by chemical reaction may be achieved by creating the necessary reaction conditions between the attachment sites and functional sites of the specimen to form a chemical bond between the attachment sites and the specimen. As another example, a mechanical interlocking mechanism involving piezoelectric actuation might require passing an electrical current through the attachment site.

Method 100 may proceed to Block 130 by mounting the specimen support in a CPM for analysis. CPMs suitable for the methods 100-600 and other methods of the disclosure include, but are not limited to, scanning electron microscopes (SEMs), transmission electron microscopes (TEMs), and STEMs. In alternative embodiments, Blocks 110 and 120 may be carried out after Block 130. In such embodiments, the specimen support is populated with specimens after mounting the specimen support in the CPM.

Method 100 may continue at Block 140 by locally scanning the plurality of pre-defined locations to determine the positions of the specimens. Locally scanning a pre-defined location may include scanning all or a portion of the pre-defined location area, scanning one or more areas adjacent and/or proximate to the pre-defined location, or a combination thereof. All of the pre-defined locations may be scanned or a portion of the pre-defined locations may be scanned. In some embodiments, scanning the pre-defined locations is terminated upon locating a certain number of specimens, upon location and identification of specific specimens, or a combination thereof.

Locally scanning the pre-defined locations may include interrogating the pre-defined locations with a charged particle beam. Scanning may be carried out with a charged particle beam at beam resolutions sufficient to determine the positions of the specimens. In some embodiments, images of the pre-defined locations are generated by interrogating the pre-defined locations with a charged particle beam at a resolution greater than or equal to a minimum resolution required to determine a position of the specimen and less than a minimum resolution required to analyze the specimen. By locating the specimens using resolutions lower than required to analyze the specimens, the time required to locate the specimens with the CPM may advantageously be reduced because CPMs require less time to generate low resolution images than for high resolution images.

Method 100 may continue to Block 150 by processing the charged particle beam data obtained in Block 140 to determine the position of the specimens on the support surface. In embodiments where images are obtained with a charged particle beam, the positions of the specimens can be determined by processing the images. In such embodiments, the locations of the specimens on the support surface can be determined, for example, by visual inspection, by pattern recognition, or a combination thereof. The positions may then be recorded to a memory, such as a hard drive, for recall in Block 160.

Method 100 may continue at Block 160 by scanning the specimens with a charged particle beam from a CPM at resolutions sufficient to extract desired information from the specimens. The locations saved in Block 150 may be used to guide the optical axis of the CPM to each specimen so that scanning may begin. Examples of desired information include, but are not limited to, information about a specimen's chemical composition, structure, morphology, or any combination thereof. In imaging applications, resolutions of the charged particle beam should be high enough to produce images of the specimens at the desired clarity. The method may then proceed to Block 170 by processing the data extracted from the specimens to obtain the desired information about the specimens. Additionally or alternatively, the processing of Block 170 may be carried out as beam data is acquired in Block 160. Likewise, the processing of Block 150 may be carried out during, after, or both before and after the scanning of Block 140. The processing steps described elsewhere in the disclosure may similarly be carried out.

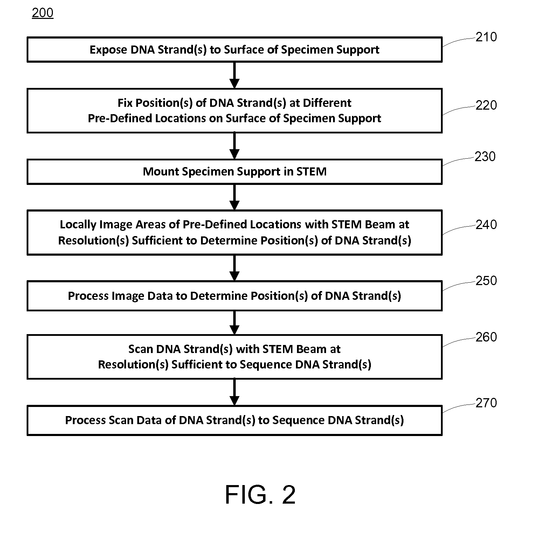

Method 100 may be carried out in a variety of applications. One such application is DNA sequencing. Referring to Figure 2, a flow diagram for a method 200 of locating and sequencing DNA on a specimen support is shown. Method 200 represents embodiments of method 100 wherein specimens comprising DNA strands are located and sequenced using a STEM. Although illustrated for single-stranded DNA, DNA sequencing embodiments may be carried out on specimens comprising single stranded DNA, double stranded DNA, or a combination thereof.

Method 200 may begin at Block 210 by exposing DNA strands to a surface of a specimen support having a plurality of pre-defined locations. The support surface may also comprise a plurality of functionalized attachment sites distributed at or proximate to each of the pre-defined locations and functionalized to fix positions of the DNA strands at the predefined locations. Exposing the DNA strands to the support surface may include, for example, bringing the DNA strands in proximity to the functionalized attachment sites to facilitate occupation of the pre-defined locations by the DNA strands. The DNA strands may be long-read DNA strands having lengths of greater than about 20,000 bases, alternatively greater than about 30,000 bases.

Method 200 continues at Block 220 by fixing positions of the DNA strands at different pre-defined locations on the support surface. Fixing the positions of the DNA strands may include fixing a position of an end of each DNA strand at a different pre-defined location of the support surface. Fixing the positions of the DNA strands may be carried out via execution of functionalization mechanisms of the attachment sites configured to fix positions of the ends of DNA strands to the pre-defined locations. For example, the attachment sites may comprise chemical groups that will form chemical bonds with chemical functional groups of end portions of the DNA strands. In some embodiments, additional steps are carried out to straighten each of the DNA strands and/or orient the DNA strands in a common pre-defined direction. DNA strands may be straightened and/or commonly aligned according to techniques developed by, for example, ZS Genetics, Inc. of Wakefield Massachusetts.

In some embodiments, a carbon substrate comprises a support surface having attachment sites located at pre-defined locations and functionalized to chemically attach to ends of the DNA strands. The carbon substrate may be a thin carbon film, which may be supported by a microscope grid. The DNA strands may remain substantially intact during the exposing, attaching, and optional orienting steps, even for DNA strands up to 30,000 bases length. Without wishing to be bound by theory, it is believed that the integrity of the DNA strands is maintained because the inter-base spacings in DNA, which are approximately 0.34 nm in length, allow for minor amounts of stretching as the DNA strands are physically manipulated.

At Block 230, the specimen support is mounted in a STEM such that the DNA strands may be interrogated by an electron beam of the STEM. Alternatively, the specimens may be exposed and affixed after the specimen support has been mounted.

Once the DNA strands are ready for interrogation by the STEM, method 200 continues to Block 240, wherein an electron beam of the STEM is used to locally image the pre-defined locations at resolutions sufficient to determine positions of the DNA strands at occupied pre-defined locations. In some embodiments, the electron beam locally images the pre-defined locations at resolutions greater than or equal to a minimum resolution required to determine a position of the DNA strand and less than a minimum resolution required to sequence the DNA strand. By locating the DNA strands using resolutions lower than required for sequencing, the time required to locate the specimens with the CPM may advantageously be reduced because CPMs require less time to generate low resolution images than for high resolution images.

At Block 250, the image data of the pre-defined locations acquired by the STEM is processed to determine the positions of the DNA strands on the support surface. The image processing of Block 250 may be carried out concurrently and/or subsequent to the scanning of Block 240. The positions may then be recorded to a memory, such as a hard drive, for recall in Block 240.

Method 200 may then continue to Block 260 by scanning the DNA strands with an electron beam of the STEM at a resolution sufficient to sequence the DNA strands.

The locations saved in Block 250 may be used to guide the optical axis of STEM to each DNA strand so that scanning of the DNA strand may begin in Block 260. At Block 270, scan data of the DNA strands acquired by the STEM is processed to determine the sequences of the DNA strands. The data processing of Block 270 may be carried out concurrently and/or subsequent to the scanning of Block 260.

In various embodiments, the method includes locally scanning by beam or imaging by light microscope the pre-defined locations to detect the presence of specimens at pre-defined locations, scanning pre-defined locations occupied by the specimens to determine positions of the specimens, and then scanning the located specimens to analyze the specimens. The located specimens may be scanned at resolutions higher than used to determine positions of the specimens, and the resolutions used to determine positions of the specimens are higher than resolutions used to detect the presence of specimens. The specimens may be labeled to facilitate locating and/or analyzing the specimens.

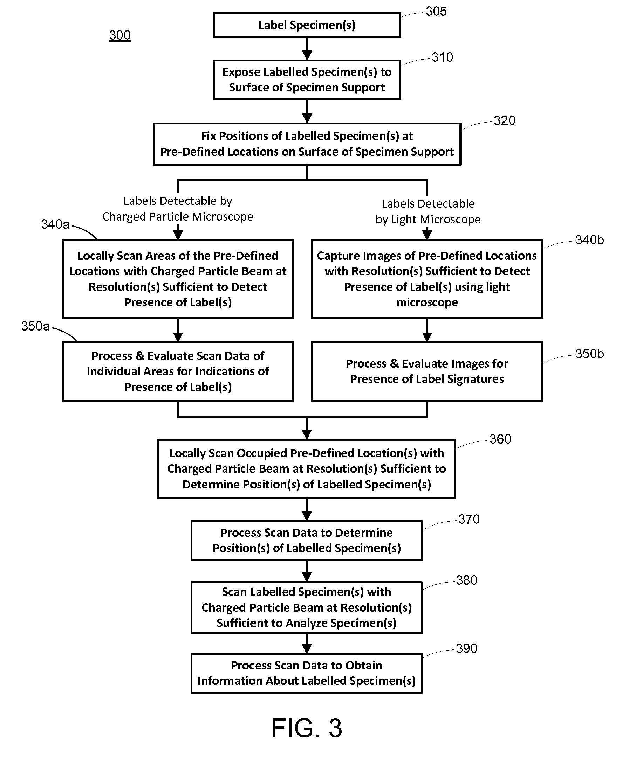

Turning now to FIG. 3, a flow diagram of a method 300 of locating and imaging specimens on a sample support according to an embodiment of the disclosure is shown. Method 300 may begin at Block 305 by labeling the specimens with labels detectable by a microscope at resolutions lower than would be required to detect the specimens in an unlabeled state. Labeling a specimen may include, for example, attaching a label structure to a specimen, modifying the structure and/or composition of a specimen so that all or part of the specimen functions as a label, or a combination thereof. The label structures may include structures and/or molecules that can be attached to individual specimens. Labelling may be carried out according to suitable techniques known in the art for the labels and specimens of a given application. For example, well-known methods exist for generating and attaching fluorescent labels to proteins specimens.

In some embodiments, the labels are configured to provide information about the specific specimens. For example, each specimen may be associated with a label that uniquely distinguishes the specimen from other specimens on a support structure. As another example, the labels may encode information about the chemical structure of a specimen. In one such example, the labels are configured to encode information about the chemical structure that can be extracted from the specimen with a microscope at resolutions lower than the specimen in an unlabeled state.

Method 300 may continue at Block 310 by exposing the labelled specimens to the surface of a specimen support and then at Block 320 by fixing positions of the labelled specimens at pre-defined locations on the surface of the specimen support. The specimen support may then be mounted in a microscope (not shown). The exposing, fixing, and mounting of method 300 may be carried out as described above with respect to Blocks 110, 120, and 130. The labeling, exposing, fixing, and mounting of method 300 may be varied, and are not limited to the order shown in Figure 3. Further, execution of one or more of the labeling, exposing, fixing, and mounting steps of method 300 may also overlap.

In some embodiments, the specimens are associated with labels that are detectable with a charged particle beam at resolutions lower than would be required to detect the presence of the specimens in an unlabeled state. For example, the labels may comprise heavy-atom tags and/or light-emitting labels detectable by a charged particle beam of a CPM at beam resolutions lower than required to detect the presence of the specimens in an unlabeled state. As another example, the labels may comprise light-emitting labels detectable with an electron beam of a CPM at beam resolutions lower than required to detect the specimens in an unlabeled state. Utilizing labels detectable at lower resolutions than required for detecting the specimens may advantageously result in faster location of the specimens because CPMs require less time to generate low resolution images than for high resolution images.

In such embodiments, method 300 may continue at Block 340a by locally scanning areas of the pre-defined locations with a charged particle beam at resolutions sufficient to detect the presence of heavy-atom tags. In one such embodiment, the beam resolutions are greater than or equal to a minimum resolution required to detect the presence of a label at a pre-defined location and less than a minimum resolution required to determine a position of a specimen at an occupied pre-defined location. In Block 350a, data for the individual pre-defined locations acquired according to Block 340a is processed and evaluated for indications of the presence of labels to identify occupied pre-defined locations.

In some embodiments, the specimens are associated with labels detectable in images captured by an optical microscope having resolutions lower than required to detect the presence of the specimens in an unlabeled state. For example, the labels may comprise light-emitting labels configured to emit light at intensities detectable in images captured by a light-optical microscope having a resolution lower than required to detect presence of the specimen in an unlabeled state.

In such embodiments, method 300 may continue at Block 340b by capturing images of the pre-defined attachment locations with resolutions sufficient to detect the presence of the specimens using a light microscope. In one such embodiment, the images have resolutions greater than or equal to a minimum resolution required to detect the presence of a label at a pre-defined location and less than a minimum resolution required to determine a position of a specimen at an occupied pre-defined location. In Block 350b, the images are processed and evaluated for indications of the presence of labels to identify occupied pre-defined locations.

From Blocks 350a and 350b, method 300 may continue at Block 360 by locally scanning the occupied pre-defined locations at beam resolutions sufficient to determine positions of the labelled specimens. In an embodiment, the beam resolutions are greater than or equal to a minimum resolution required to determine positions of the specimens at the occupied pre-defined locations and less than a minimum resolution required to analyze the specimens. By locating the specimens using resolutions lower than required to analyze the specimens, the time required to locate the specimens with the CPM may advantageously be reduced because CPMs require less time to generate low resolution images than for high resolution images.

In Block 370, beam data acquired according to Block 360 is processed to determine the positions of the labelled specimens. The positions of the specimens may then be recorded to a memory, such as a hard drive, for recall in Block 380. The locations saved in Block 370 may be used to guide the optical axis of the charged particle apparatus to each specimen so that scanning of the specimen may begin.

Method 300 may continue at Block 380 by scanning the individual labelled specimens with a charged particle beam at resolutions sufficient to analyze the specimens. The charged particle beams of Blocks 340a, 360, and 380 may be generated from the same CPM or different CPMs. In Block 390, the scan data obtained according to Block 380 is processed to obtain information about the labelled specimens.

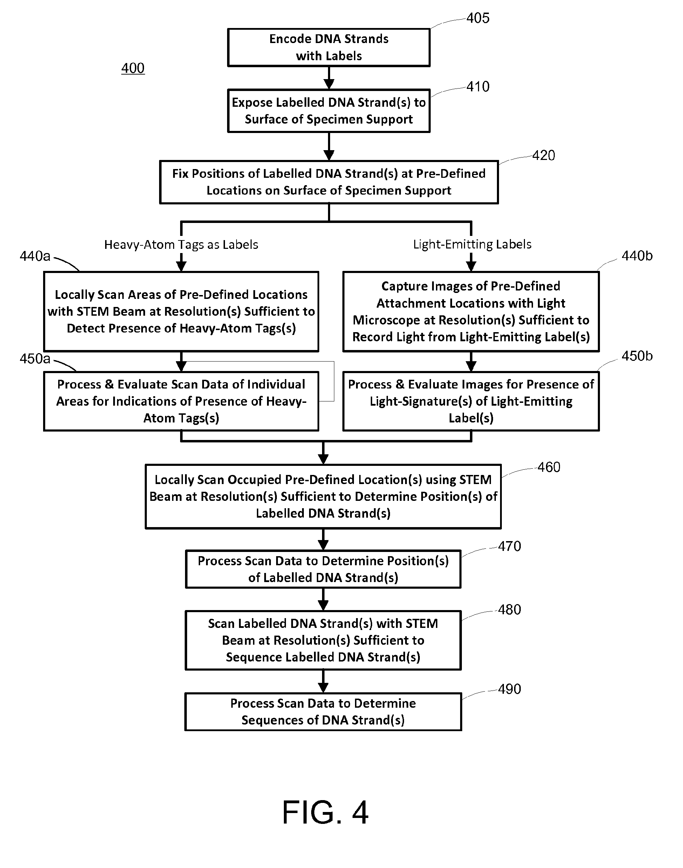

Method 300 may be carried out in a variety of applications. One such application is DNA sequencing. Referring to Figure 4, a flow diagram for a method 400 of locating and sequencing labelled DNA on a specimen support is shown. Method 400 represents embodiments of method 300 wherein specimens comprising DNA strands are labelled, located, and sequenced using a STEM.

Method 400 may begin at Block 405 by encoding DNA strands with labels detectable by microscope at resolutions lower than would be required to detect the specimens in an unlabeled state. The DNA strands may be long-read DNA strands having lengths of greater than about 20,000 bases, alternatively greater than about 30,000 bases.

In some embodiments, the DNA strands are encoded by attaching heavy-atom tags (*e.g.,* Hg) detectable by an STEM at resolutions lower than required to detect unlabeled DNA strands. Heavy-atom tags may be attached directly to the bases of the DNA strands, for example, during the DNA polymerase reaction that converts the DNA from single-stranded to double-stranded. The bases of the DNA strands may be tagged sequentially, intermittently, or a combination thereof. In some embodiments, the adenine, thymine, cytosine, and guanine bases of the DNA strands are labeled with different species of heavy-atom tags. When imaged by a STEM, each species of heavy-atom tag produces a signal having a characteristic intensity, which can be distinguished in the resulting image of the labeled DNA strand. Thus, the DNA strands can be sequenced from the manifestations of the heavy-atom tags in images of the DNA strands captured by an STEM. Additionally or alternatively, the DNA strands may be labelled with heavy-atom tags such that bases and/or sequences of bases may be indicated by gaps in sequences of heavy-atom tags. Labeling the DNA strands with heavy-atom tags may be carried out according to suitable techniques known in the art, such as those developed by the University of Chicago and refined by ZS Genetics, Inc., of Wakefield, Massachusetts.

In various embodiments, the DNA strands are labeled with light-emitting labels that emit a light signature detectable by STEM at resolutions lower than required to detect the DNA strands in an unlabeled state. Light-emitting labels can typically be detected by STEM using imaging scans much coarser than required for DNA sequencing. In some embodiments, the DNA strands are associated with light-emitting labels that emit a light signature detectable by light-optical microscope. The light-emitting labels may additionally contain encoded information relating to the source of the DNA strand. Labeling the DNA strands with light-emitting labels may be carried out according to suitable techniques known in the art.

Method 400 may continue at Block 410 by exposing the labelled DNA strand to a functionalized surface of a specimen support, and then at Block 420 by fixing the labelled DNA strands at pre-defined locations on the surface of the specimen support. The specimen support may then be mounted in a microscope (not shown). The encoding, exposing, fixing, and mounting of method 400 may be carried out as described above with respect to Blocks 110, 120, and 130.

The labeling, exposing, fixing, and mounting of method 400 may be varied, and are not limited to the order shown in Figure 4. Further, execution of one or more of the labeling, exposing, fixing, and mounting steps of method 400 may also overlap.

In some embodiments, a carbon substrate comprises a surface having attachment sites located at pre-defined locations and functionalized to chemically attach to ends of the labelled DNA strands. The carbon substrate may be a thin carbon film, which may be supported by a microscope grid. The labelled DNA strands may remain substantially intact during the labeling, exposing, attaching, and optional orienting steps, even for DNA strands up to 30,000 bases length. Without wishing to be bound by theory, it is believed that the integrity of the DNA strands is maintained because the inter-base spacings in DNA, which are approximately 0.34 nm in length, allow for minor amounts of stretching as the DNA strands are physically manipulated.

In embodiments involving heavy-atom tagged DNA, method 400 may continue at Block 440a by locally scanning areas of the pre-defined locations with an electron beam of a STEM at beam resolutions sufficient to detect the presence of heavy-atom tags. In an embodiment, the beam resolutions are greater than or equal to a minimum resolution required to detect the presence of heavy-atom tags at a pre-defined location and less than a minimum resolution required to determine a position of a DNA strand at an occupied pre-defined location. For example, the STEM may be used to generate "fine" images of relatively small pixels having sizes in a range of about 1 nm to about 2 nm. Such fine images are typically inadequate for sequencing DNA strands directly and for sequencing DNA strands using the heavy-atom tags, which typically requires "sequencing" images comprising pixel sizes of less than about 0.4 nm. However, fine images of the heavy-atom tags are sufficient to detect the presence or absence of a strand at each pre-defined location, which may prevent unnecessary higher resolution scanning of un-occupied locations when locating specific positions of the DNA strands with the STEM. Using lower resolution scanning to identify which pre-defined locations are occupied (e.g., Blocks 340a, 340b, 440a, and 440b) may result in significant time savings in situations where, for example, the occupancy ratio of the populated specimen attachment locations is low. As used herein, the "occupancy ratio" refers to the ratio of the number of specimens to pre-defined locations. The occupancy ratio is an indicator of the likelihood that a pre-defined location will be unoccupied when initially scanned with a charged particle beam.

In Block 450a, data for the individual pre-defined locations acquired according to Block 440a is processed and evaluated for indications of the presence of labels to identify occupied pre-defined locations. Heavy-atom tags can be detected by STEM using imaging scans much coarser than required for DNA sequencing. As such, utilizing heavy-atom tags to determine which of the pre-defined resolutions are occupied by DNA strands may advantageously result in faster location of the DNA strands because the STEM requires less time to generate low resolution images than for high resolution images. Steps 440a and 450a may also be carried out on DNA strands labeled with heavy-atom tags detectable by a STEM electron beam at beam resolutions lower than required to detect the DNA strands in an unlabeled state.

In embodiments where the labels emit light detectable in optical microscope images, method 400 may continue at Block 440b by using a light microscope to capture images of the pre-defined attachment locations having resolutions sufficient to detect light signatures of the light-emitting labels. In an embodiment, the images have resolutions greater than or equal to a minimum resolution required to detect the light signatures and less than a minimum resolution required to determine the position of a DNA strand occupying one of the pre-defined locations. In Block 450b, the images of the individual pre-defined locations are processed and evaluated for the presence of the light signatures to identify occupied pre-defined locations.

From Blocks 450a and 450b, method 400 may continue at Block 460 by locally scanning the occupied pre-defined locations with an electron beam of a STEM having beam resolutions sufficient to determine positions of the labeled DNA strands. In an embodiment, the beam resolutions are greater than or equal to a minimum resolution required to determine positions of the DNA strands at the occupied pre-defined locations and less than a minimum resolution required to sequence the DNA strands. By locating the DNA strands using resolutions lower than required for sequencing, the time required to locate the specimens with the CPM may advantageously be reduced because CPMs require less time to generate low resolution images than for high resolution images. In Block 470, beam data acquired according to Block 460 is processed to determine the positions of the labeled DNA strands. The positions may then be recorded to a memory, such as a hard drive, for recall in Block 480.

Method 400 may continue at Block 480 by scanning the individual labelled DNA strands with an electron beam of an STEM at resolutions sufficient to analyze the DNA strands. The locations saved in Block 470 may be used to guide the optical axis of the STEM to each DNA strand so that scanning of the DNA strand may begin. The electron beams of Blocks 440a, 460, and 480 may be generated from the same or a different STEM. In an embodiment, the DNA strands are scanned at resolutions sufficient to sequence the heavy-atom tags and less than a minimum resolution required to directly sequence the DNA.

In Block 490, the DNA scans are sequenced by processing the data obtained according to Block 480. In embodiments where bases of the DNA strands are encoded with specific species of heavy-atom tags, the labeled DNA strands may be sequenced from the STEM images as described above.

As illustrated in FIGS. 1-4, embodiments of the method for locating specimens involve fixing positions of specimens at different pre-defined locations on the sample surface. By limiting where the specimens may be found to the pre-defined locations, the total search area required for locating all of the specimens is reduced. Such reductions may in turn result in substantial time savings compared to scanning times achieved by CPMs using conventional rastering techniques where the whole support surface may be scanned and the total scanning time depends directly on the total size of the area to be scanned. Thus, by fixing specimens to pre-defined locations and limiting scanning to areas corresponding to pre-defined locations, methods 100-400 may provide rapid location of extremely small specimens on relatively large supports.

In some embodiments, labeled specimens are located on a surface of a specimen support without relying on pre-defined locations. In such embodiments, a portion of the support surface containing a plurality of specimens may be scanned by charged particle beam or imaged by light microscope at resolutions sufficient to determine the approximate positions of labels associated with the specimens. The resolutions may be less than a minimum resolution required to detect the positions of specimens in an unlabeled state. The support surface may then be locally scanned at areas encompassing the approximate positions of the labels at resolutions sufficient to determine the positions of the labelled specimens, after which the labelled specimens are scanned by charged particle beam at resolutions sufficient to analyze the specimens.

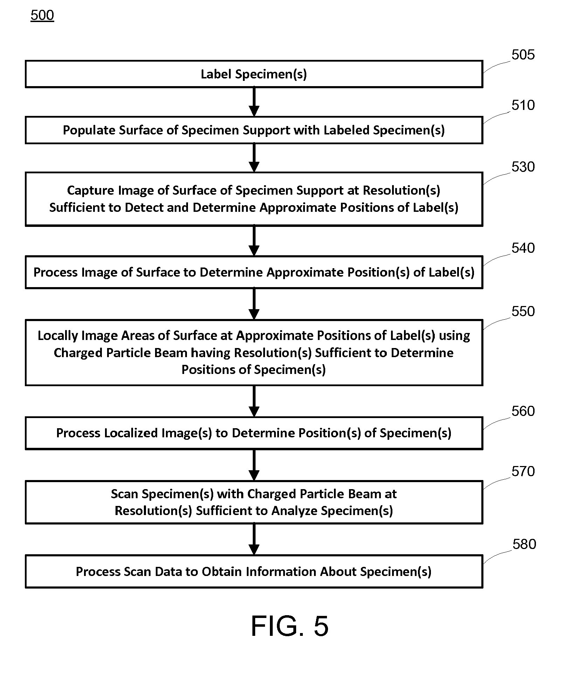

Referring to FIG. 5, a flow diagram for a method 500 of locating and imaging labeled specimens on a specimen support in accordance with another embodiment is shown. The method may begin at Block 505 by labeling the specimens with labels that may be located at resolutions less than required to locate the specimens in an unlabeled state. Suitable labels include, but are not limited to, light-emitting labels and heavy-atom tags. The method of labelling described in connection with block 305 is suitable for Block 505.

Method 500 continues at Block 510 by populating the surface of the specimen support with the labeled specimens. The surface may comprise functionalized attachment sites distributed at different locations on the surface. In such cases, the exposing and fixing steps described for Blocks 310 and 320, respectively, may be used to populate the surface with specimens in Block 510. Additionally or alternatively, specimens may be distributed over the support surface at different locations and held in place by gravity and/or chemical forces.

Method 500 continues at Block 530 by capturing an image of the surface of the specimen support at resolutions sufficient to detect and determine approximate positions of the specimen labels. In an embodiment, the image of the surface has a resolution greater than a minimum resolution required to determine approximate positions of the labels and less than a minimum resolution required to determine sufficient to determine positions of the specimens on the support surface. Imaging may be carried out with a CPM, an optical microscope, or a combination thereof. At Block 540, the method continues by processing the image of the surface to determine the approximate positions of the labels.

Method 500 continues at Block 550 by locally imaging areas of the surface at the approximate positions of the label(s) using a charged particle beam having resolutions sufficient to determine positions of the specimens. That is, each captured image corresponds to a localized area of the surface containing one of the approximate positions of the labels. In an embodiment, the local images are captured at resolutions greater than or equal to a resolution sufficient to determine positions of the specimens and less than resolutions required to image the specimen. By locating the specimens using resolutions lower than required to analyze the specimens, the time required to locate the specimens with the CPM may advantageously be reduced because CPMs require less time to generate low resolution images than for high resolution images.

At Block 560, the local images are processed to determine positions of the specimens on the surface. The positions of the specimens may then be recorded to a memory, such as a hard drive, so that they may be recalled in Block 570 for proper placement of the charged particle beam.

Method 500 continues at Block 570 by scanning the specimens with a charged particle beam at resolutions sufficient to analyze the specimen, and then at Block 580 by processing scan data of the charged particle beam to obtain information about the specimens. The locations saved in Block 560 may be used to guide the optical axis of the charged particle apparatus to each specimen so that scanning of the specimen may begin. Blocks 570 and 580 may be carried out, for example, as described above for Blocks 370 and 380.

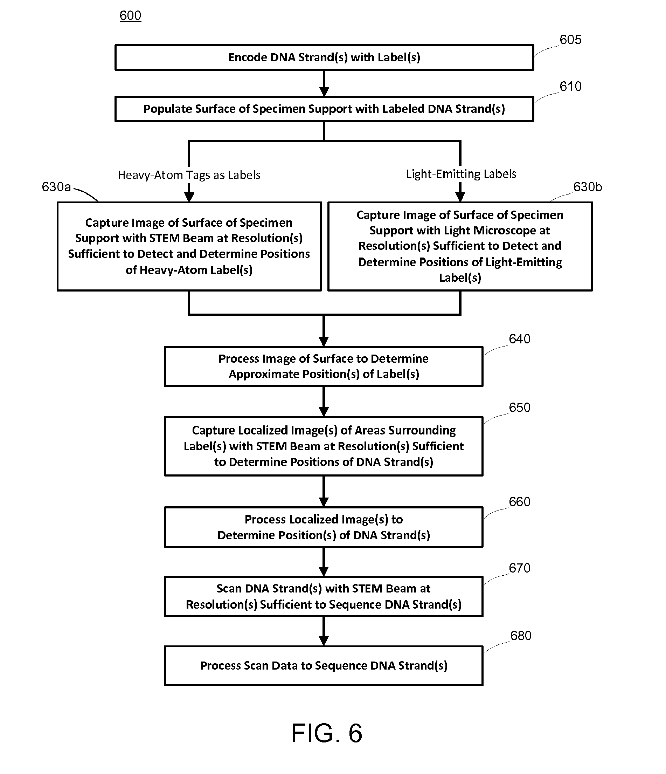

Method 500 may be carried out in a variety of applications. One such application is DNA sequencing. Referring to Figure 6, a flow diagram for a method 600 of locating and sequencing labelled DNA on a specimen support is shown. Method 600 represents embodiments of method 300 wherein specimens comprising DNA strands are labelled, located, and sequenced using a STEM.

Method 600 begins at Block 605 by encoding DNA strands with labels locatable at resolutions lower than required to locate the specimens in an unlabeled state. Suitable labels include, but are not limited to, light-emitting labels and heavy-atom tags. Heavy-atom tags can typically be located by STEM using imaging scans coarser than required for locating DNA strands. The DNA strands may be encoded with labels in Block 605, for example, as described above in connection with block 405.

Method 600 continues at Block 610 by populating a support surface of a specimen support with the labeled DNA strands. In such cases, the exposing and fixing steps described for Blocks 410 and 420, respectively, may be used to populate the surface with the labelled DNA strands in Block 610. In an embodiment, the support surface comprises a carbon substrate comprising functionalized attachment sites distributed over the surface at different locations. The carbon substrate may be a carbon film supported by a microscope grid. In an alternative embodiment, the DNA strands are distributed over the support surface at different locations and held in place by gravity and/or chemical forces.

For DNA strands labelled with heavy-atom tags, the method may continue at Block 630a by capturing imaging the surface of the specimen support at resolutions sufficient to detect and determine positions of the heavy-atom labels. Step 630a may also be carried out on DNA strands labeled with light-emitting labels detectable by a STEM electron beam at beam resolutions lower than required to detect the DNA strands in an unlabeled state. Surfaces populated by DNA strands labeled with light-emitting labels may alternatively be imaged according to Block 630b, where a light microscope is used to detect and determine the positions of the light-emitting labels. In an embodiment, an image acquired according to Block 630a or Block 630b has a resolution greater than a minimum resolution required to determine approximate positions of the labels and less than a minimum resolution required to determine positions of the specimens on the support surface. The images captured in Blocks 630a, 630b are then processed at Block 640 to determine the approximate positions of the labels.

Method 600 continues at Block 650 by locally imaging areas of the surface at the approximate positions of the label(s) using a STEM electron beam with a resolution sufficient to determine positions of the DNA strands. At Block 660 the local images are processed to determine the positions of the labelled DNA strands on the support surface. The approximate label positions may be imaged at Block 650 in the same manner described for Block 450 with respect to the pre-defined locations. The images obtained at Block 650 may be processed in the manner described for Block 460. The positions of the DNA strands may then be recorded in Block 660 to a memory, such as a hard drive, so that they may be recalled in Block 670 for proper placement of the electron beam of the STEM.

Approximate locations of the specimens may be determined rapidly at Block 630a even though an area large enough to include all of the labelled specimens is imaged. This is because the labels may be scanned using a much coarser raster (i.e., larger pixels) to resolve approximate locations of the specimens since signals from the labels will be either UV or visible light, or large heavy-atom labels. Thus, a coarse image of an area including all of the samples may be acquired rapidly at Block 630a and then processed in Block 640 to approximately locate the DNA strands. Areas of the approximate locations are then imaged at fine pixel sizes at Blocks 650 and 660 to determine the actual positions of the DNA strands.

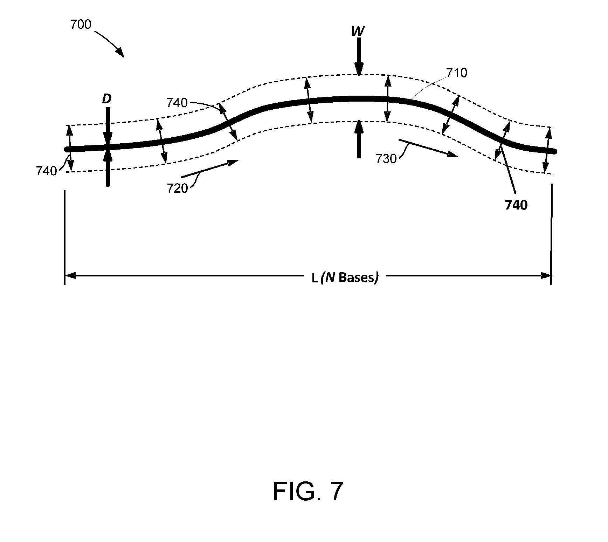

After the positions of the DNA strands have been determined, method 600 continues at Block 670 by scanning the DNA strands with a STEM electron beam at resolutions sufficient to sequence the DNA strands, and then at Block 680 by processing the electron beam data to sequence the DNA strands. The locations saved in Block 660 may be used to guide the optical axis of the STEM to each DNA strand so that scanning of the DNA strand may begin. Blocks 670 and 680 may be carried out, for example, as described above for Blocks 470 and 480. Once all the DNA locations have been determined, the very high resolution imaging process ("sequencing" image) illustrated in FIGS. 7-9 and Table I is performed for DNA sequencing.

After locations of the DNA strands have been determined in methods 200, 400, and 600, an efficient beam strategy may be utilized to carry out sequencing at Blocks 260, 480, and 670. An embodiment of the efficient beam scanning strategy is illustrated in FIGS. 7-9.

FIG. 7 shows a possible efficient imaging strategy for a typical long-read DNA strand 700 of length L, diameter D, and a number of bases N that may be processed according to embodiments of the disclosure. The long-read DNA strand 700, for example, may have a diameter $D$ of approximately 2 nm and a number of bases N of about 30,000, each of the bases being spaced apart about 0.34 nm away from one another. With minor amounts of stretching, the long-read DNA strand 700 may be deposited on a support surface in generally straight form (*e.g.*, no sharp bends) and at an overall length $L$ of approximately 12 $\mu$m. Long-read DNA strand 700 may then be interrogated by an electron beam of a STEM according to a sequence of fast line scans that are executed in sequential fashion along axis 710 of long-read DNA strand 700 in the directions of arrows 720 and 730. Each of the fast line scans run perpendicular to the local directions of axis 710 along axes 740 as shown for lengths corresponding to scan width W. Lengths of the scan lines and the scan width W may be, for example, about 10 nm.

For maximum use of pixels, a dynamic scan strategy may be utilized where the total area scanned is:

$$(10 \text{ nm}) \times (12000 \text{ nm}) = 120000 \text{ nm}^2 = 0.12 \text{ } \mu\text{m}^2 = (0.35 \text{ } \mu\text{m})^2.$$

With pixels of size d (in nm), the number of pixels would be:

$$(120000 \text{ nm}^2)/d^2.$$

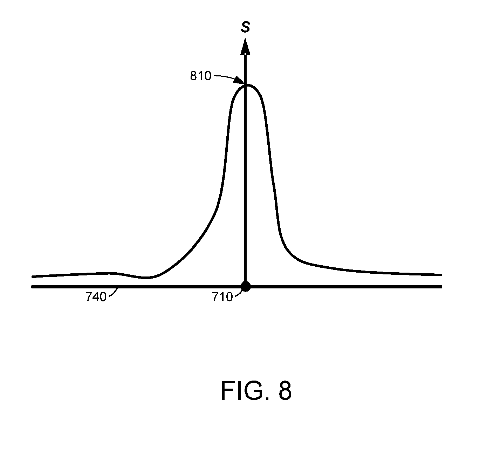

FIG. 8 shows the strength of signal S of the electron beam across one of the 10 nm-wide fast scan axes 740 shown in FIG. 7. Axis 710 of long-read DNA strand 700 runs normal to the plane of FIG. 8 and is represented by the dot at the intersection of the axis of signal strength S and fast scan axis 740. When the STEM centers the scan on long-read DNA strand 700, then the signal peak 810 will also be centered as shown. For a 10 nm scan, the 2 nm DNA strand will represent about 20% to about 30% of the scan width.

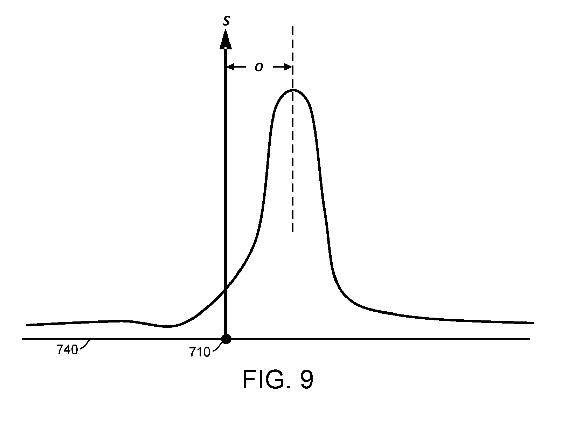

FIG. 9 shows signal S when the scan has drifted off the center of the 2 nm DNA strand by an offset O. This situation may arise, for example, either from bending of long-read DNA strand 700 strand and/or from instabilities in the scan electronics or column mechanical structure. Once the peak of the scan signal is off-center (to the right in this example) the scan electronics of the STEM may compensate by also moving the scan to the right, thereby tracking the DNA scan as the STEM images along the entire length of the long-read DNA strand 700. As the DNA strand bends around on the substrate, the orientation of the fast scan axis may be adjusted to maintain the scan axis perpendicular to the local direction (*e.g.,* arrows 720, 730) of the DNA strand 700. Table I below shows the calculated number of pixels and imaging time (at 10 MHz) for various pixel sizes. However, if sequencing times are the dominant consideration for $3^{rd}$-generation sequencing, then one or more of the three embodiments of the present invention may be preferred. The total imaging times represent the execution times of Blocks 260, 480, or 670.

TABLE. I. As a function of the pixel size, the total numbers of pixels, the imaging time, and the sequencing rate are illustrated here.

| Pixel Size | # Pixels in $12 \times 10^4$ nm$^2$ | Total Imaging Time at 10 MHz | Sequencing Rate |
|---|---|---|---|
| (nm) | | (s) | (bases/hour) |
| 1.000 | 1.20E+05 | 0.012 | 9.00E+09 |
| 0.500 | 4.80E+05 | 0.048 | 2.25E+09 |
| 0.250 | 1.92E+06 | 0.192 | 5.63E+08 |
| 0.125 | 7.68E+06 | 0.768 | 1.41E+08 |
| 0.100 | 1.20E+07 | 1.200 | 9.00E+07 |
| 0.080 | 1.88E+07 | 1.875 | 5.76E+07 |
| 0.060 | 3.33E+07 | 3.333 | 3.24E+07 |
| 0.050 | 4.80E+07 | 4.800 | 2.25E+07 |
| 0.040 | 7.50E+07 | 7.500 | 1.44E+07 |
| 0.030 | 1.33E+08 | 13.333 | 8.10E+06 |
| 0.020 | 3.00E+08 | 30.000 | 3.60E+06 |
| 0.015 | 5.33E+08 | 53.333 | 2.03E+06 |

Also disclosed herein are functionalized specimen supports that may be useful for finding and locating small specimens distributed on a relatively large surface of the functionalized specimen support. The functionalized specimen supports may facilitate efficient location of specimens when used in accordance with one or more of methods 100 through 600 of FIGS. 1-6.

The functionalized specimen support comprises a surface for supporting specimens during microscopy applications. The support surface is configured to support specimens during an interrogation of the specimens with a CPM. The support surface includes functionalized sites distributed at different locations on the support surface. Each functionalized site is configured to maintain a position of a specimen at the location of the functionalized site. The functionalized sites may maintain the positions of the specimens by way of attachment, attraction, or a combination thereof.

The attachment sites may be functionalized to fix the positions of specimens according to one or more mechanisms. Suitable mechanisms include, but are not limited to, chemical attachment (*e.g.,* a bond-forming chemical reaction between the attachment site and the specimen), exertion of an attractive force (*e.g.,* exertion of an electrostatic field opposite in sign to an electrostatic charge of the specimen; intermolecular forces between molecules of the attachment site and the specimen), adhesion, and mechanical engagement (*e.g.,* via a nanoscale interlocking mechanism configured to fixedly engage a specimen).

The attachments sites may be configured to exert an attractive force on the specimens when the specimens come within a proximity of the attachment sites. For example, attachment sites having a positively charged surface may be used in a functionalized specimen support in an application involving the analysis of specimens having negatively charged surfaces. A negatively charged specimen would be attracted to one of the positively charged attachment sites if exposed to the positively charged attachment site at a proximity sufficient for the electrostatic forces to take hold.

The attachment sites may also be configured to attach specimens brought within proximity to the attachment sites upon the creation of conditions necessary to trigger attachment mechanisms of the attachment sites. For example, an attachment site may comprise one or more chemical functional groups that covalently bond with a specimen in the presence of certain reactants and at certain temperatures and pressures. As another example, a mechanical interlocking mechanism involving piezoelectric actuation might require passing an electrical current through the attachment site.

In various embodiments, the attachment sites occupy and/or are proximate to a plurality of pre-defined locations on the support surface. A pre-defined location refers to a location on a surface of a specimen support that is known prior to use of the specimen support for locating specimens. For example, coordinates for pre-defined locations of a support surface may be stored in a machine-readable memory that is separate from the functionalized specimen support. The machine-readable memory may be a format from which the pre-defined coordinates may be readily transferred to or accessed by a system configured to process specimens supported by the functionalized specimen support. Examples of suitable machine-readable memory formats include, but are not limited to, CDs, DVDs, portable flash drives, cloud-accessible hard-drives, and the like. The machine-readable memory may also be a memory of a CPM system configured to access the pre-defined locations in conjunction with executable instructions for carrying out one or more methods 100-600.

As another example, the coordinates of the pre-defined locations may be physically integrated into the functionalized specimen support in a form that facilities easy identification of the pre-defined locations on the support surface. In an exemplary embodiment, the functionalized specimen support takes the form of a "finder grid," which comprising letters and/or other markings etched into the grid next to pre-defined locations.

A pre-defined location may be configured for concurrent occupation by a specific number or maximum number of specimens at a given time. In an embodiment, the attachment sites of the support surface are configured such that each pre-defined location can be occupied by a single specimen at a time. Different pre-defined locations of a support surface may be configured to maintain the position of different types of specimens. In an embodiment, the functionalized specimen support comprises pre-defined locations configured to maintain a position of a first type of specimen and pre-defined locations configured to maintain a position of a second type of specimen.

The support surface of the functionalized specimen support may be a surface of the support structure. For example, the functionalized specimen support may be a surface of a TEM grid adapted to include a plurality of functionalized sites.

Alternatively, the support surface may be a surface of a film, such as a carbon film, supported by the support structure. In various embodiments, the support structure comprises a thin carbon film. Thicknesses of thin films may be in a range of from about 0.5 nm to about 10 nm, alternatively in a range of from about 0.5 nm to about 5 nm, alternatively in a range of from about 1 nm to about 2 nm. In various embodiments, the functionalized specimen support comprises a thin film comprising a plurality of functionalized attachment sites and supported by a grid structure, such as a TEM grid. The arrangement of the pre-defined attachment locations on the support surface, the number of pre-defined attachment locations, and the number and arrangement of attachment sites on the support surface may be varied as desired and/or tailored to particular applications.

The support structure may be any structure suitable for directly supporting specimens and/or supporting a structure populated with specimens in CPM applications. In various embodiments, the support structure comprises a grid structure. In one embodiment, a functionalized thin film support surface is supported by a separate commercially available TEM grid structure.

Also disclosed herein is a specimen preparation comprising a specimen support and a plurality of specimens, each of the specimens being affixed to a surface of the specimen support at one of a plurality of functionalized attachment sites and distributed at different locations on the surface. The specimen preparation may comprise any of the specimen supports disclosed herein and the specimens may be biological specimens, such as DNA strands. Embodiments of the specimen preparations may facilitate efficient location of the attached specimens when used in accordance with one or more of methods 100 through 600 of FIGS. 1-6.

Various embodiments of the specimen preparation are essentially functionalized specimen supports pre-populated with labeled and/or unlabeled specimens. In some embodiments, the specimen preparation further includes a readable non-transitory storage medium separate from the pre-populated specimen support and containing coordinates for each of the attachment sites. The storage medium may then be accessed by a CPM system and used to direct the CPM to the pre-defined locations so that the pre-defined locations may be scanned for the presence of labels and/or the positions of specimens at the pre-defined locations. In some embodiments, the functionalized specimen support of the specimen preparation comprises a finder grid, and latters etched into the grid mesh of the finder grid indicate the position of individual pre-defined locations to scan for specimens. In some embodiments, an occupancy ratio of the pre-defined locations of the specimen preparation is about 1.0. In some embodiments, an occupancy ratio of the pre-defined locations is less than about 0.75, alternatively less than about 0.50, alternatively less than about 0.25.

The arrangement of the pre-defined attachment locations on the support surface, the number of pre-defined attachment locations, and the number and arrangement of attachment sites on the support surface may be varied as desired and/or tailored for particular applications. Likewise, the labels and the method of labeling the specimens may be selected as desired and/or tailored for particular applications.

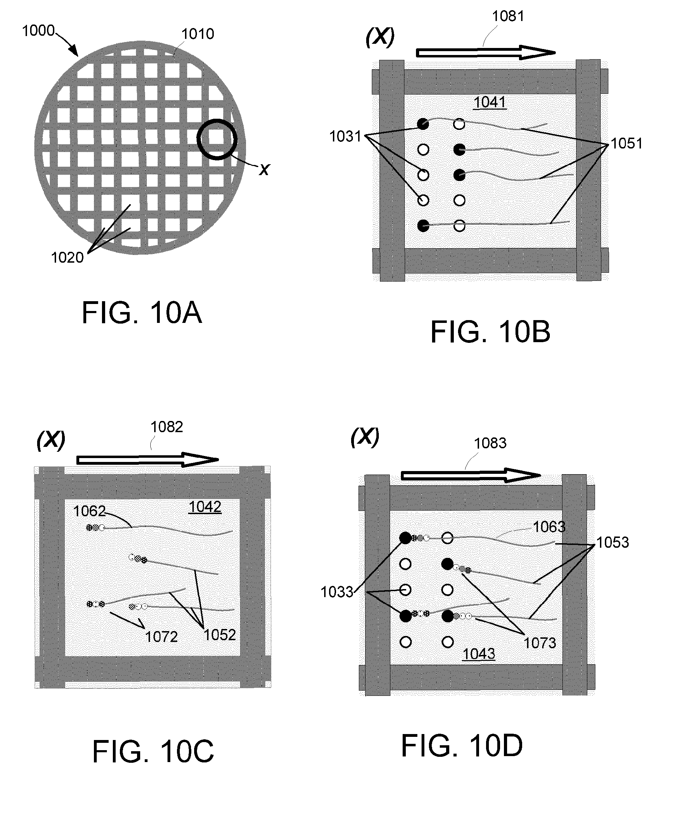

Various embodiments of the functionalized supports and specimen supports of the disclosure are illustrated in FIGS. 10A-13C. Although DNA strands and STEM-based DNA sequencing are used to illustrate FIGS. 10A-13C, the embodiments of FIGS. 10A-13C may be configured for biological specimens and/or other forms of charged-particle microscopy. Various embodiments of FIGS. 10A-13C may also be used in conjunction with light-optical microscopes, for example as described in connection with methods 300-600.

Turning to FIG. 10A, a simplified schematic illustration of a specimen support 1000 having a grid support structure 1010 comprising square openings 1020 is shown. In some embodiments, grid support structure 1010 is a microscope grid. A diameter of the microscope grid may be a standard microscope grid diameter, such as 3 mm. Microscope grids are denoted by "mesh" size, corresponding to the number of grid lines per linear inch across the grid. Thus, a "1000-mesh" grid will have 25.4 $\mu$m center-to-center spacings. Taking into consideration the widths of the mesh lines, the openings in a 1000-mesh grid may typically range from 15-20 $\mu$m (in both X-Y), comparable to the length of the 30000 base DNA strand. In such instances, one or more DNA strands may end up stretched across grid lines, which may prevent full imaging of the trespassing DNA strands in STEM-based sequencing since the electron beam of the STEM cannot penetrate through the grid lines. A carbon film, such as a thin carbon film having a thickness of about 1 nm, extends across the grid openings to provide continuous support for the DNA strands. In embodiments, the need for sufficiently wide openings to image full 12 $\mu$m DNA strands is balanced against the maximum opening over which the self-supporting 1 nm thick carbon film will not break. Suitable mesh sizes for long-read DNA strands having lengths of up to about 12 $\mu$m are in a range of from about 400 to about 1000.

FIG. 10B illustrates a close-up of a single square grid opening X of FIG. 10A in an embodiment of specimen support 1000 where pre-defined locations 1031 are provided on a support film 1041, which is supported by grid support structure 1010. DNA strands 1051 are shown. Attachment sites (not shown) within or proximate to each of pre-defined locations 1031 are functionalized to attach the DNA strands 1051 to support film 1041 at different pre-defined locations 1031. Pre-defined locations 1031 are shown as solid to indicate the presence of an attached DNA strand 1051 and unpopulated attachment sites 1031 are shown as hollow. These attachment sites may be functionalized, for example, according to well-known nano-patterning processes. Embodiments of the functionalized specimen support 1000 configured according to FIG. 10B may be used, for example, as specimens supports in method 200 described above.

FIG. 10C is a close-up of square grid opening X in an embodiment of specimen support 1000 without pre-defined attachment sites. Labeled DNA strands 1062 are randomly located on support film 1042 and comprise DNA strands 1052 and comprise labels 1072. In an embodiment, DNA strands 1052 are affixed to support film 1042 by attachment sites. In an alternative embodiment, DNA strands 1052 are maintained on support film 1042 by gravity and/or chemical forces. The labels 1072 may contain encoded information pertaining to the DNA strand 1052 to which labels 1072 are attached. These labels 1072 and the encoded data are illustrated as multiple, differently-shaded circles. The labels 1072 may be light-emitting labels and attached to DNA strands 1052 according to well-known methods for attaching light-emitting labels to DNA strands. Alternatively, heavy-atom labels with binary- or bar-codes may be employed. These heavy-atom labels would differ from the heavy-atom tags in that they would comprise much larger balls of many high-atomic number atoms which could be imaged with much larger pixels than would be necessary for DNA sequencing where single heavy atoms must be imaged. Embodiments of the functionalized specimen support 1000 configured according to FIG. 10C may be used, for example, as specimen supports in method 600 described above.

FIG. 10D is a close-up of square grid opening X in an embodiment of specimen support 1000 comprising pre-defined locations 1033 and labeled DNA molecules 1063. Attachment sites (not shown) within or proximate to each of pre-defined locations 1033 are functionalized to attach the DNA strands 1053 to support film 1043 at different pre-defined locations 1033. Labels 1073 of labeled DNA molecules 1063 may be the same as labeled molecules 1063, except that labeled molecules 1063 are affixed to support film 1043 at pre-defined locations 1033. Embodiments of the functionalized specimen support 1000 configured according to FIG. 10D may be used, for example, as specimen supports in method 400 described above. Methods 200 and 400 may advantageously use the restriction of the locations of DNA strands to specific parts of the support surface provided by the specimen supports of FIGS. 10B and 10D, respectively to locate the DNA strands faster.

The DNA strands in FIGS. 10B, 10C, and 10D may generally be oriented in a single direction. For example, the DNA strands in FIGS. 10B, 10C, and 10D may generally be oriented in the direction of arrows 1081, 1082, and 1083, respectively. Note also that although the thin films of FIGS. 10B-10D, 11B-11D and 12B-12C appear behind their respective grid squares and rectangles they are actually in front of it. The films are shown behind only to make it easier to see various features in the figures.

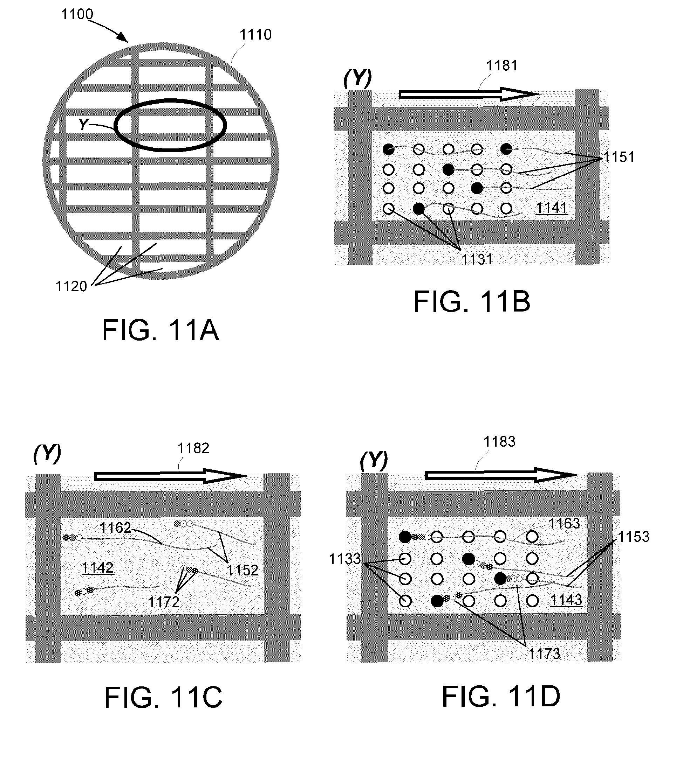

Turning to FIG. 11A, a simplified schematic illustration of a grid support structure 1110 comprising custom rectangular openings 1120, where the process for laying down generally straight DNA strands is oriented along the long axes of the rectangular openings. In some embodiments, grid support structure 1110 is a microscope grid. A diameter of the microscope grid may be a standard microscope grid diameter, such as 3 mm. As in FIG. 10A, one or more DNA strands may end up stretched across grid lines. However, the narrow dimensions of the rectangles may provide improved support for thin films supported thereon. A benefit of this custom grid (part of the present invention) compared with square conventional grids is that a potentially higher density of attached DNA strands which do not overlap grid lines may be provided in the grid area. DNA strands which overlap grid lines cannot be sequenced since there is no way to do STEM imaging when the grid line blocks transmission of electrons to the bright field and/or dark field detectors below the specimen. Thus, various embodiments of the disclosure comprise grid support structures having rectangular openings, which may advantageously increase the fraction of DNA strands which are not over grid lines and can thus be sequenced. The dimensions of the rectangular grids may be in a range of from about 25 $\mu$m to about 100 $\mu$m.

FIG. 11B illustrates a close-up of a single rectangular grid opening X of FIG. 11A in an embodiment of specimen support 1100 where pre-defined locations 1131 are provided on a support film 1141, which is supported by grid support structure 1110. DNA strands 1151 are shown. Attachment sites (not shown) within or proximate to each of pre-defined locations 1131 are functionalized to attach the DNA strands 1151 to support film 1141 at different pre-defined locations 1151. Pre-defined locations 1131 are shown as solid to indicate the presence of an attached DNA strand 1151 and unpopulated attachment sites 1131 are shown as hollow. These attachment sites may be functionalized, for example, according to well-known nano-patterning processes. Embodiments of the functionalized specimen support 1100 configured according to FIG. 11B may be used, for example, as specimen supports in method 200 described above.

FIG. 11C is a close-up of rectangular grid opening X in an embodiment of specimen support 1100 without pre-defined attachment sites. Labeled DNA strands 1162 are randomly located on support film 1142 and comprise DNA strands 1152 and comprise labels 1172. In an embodiment, DNA strands 1152 are affixed to support film 1142 by attachment sites. In an alternative embodiment, DNA strands 1152 are maintained on support film 1142 by gravity and/or chemical forces. The labels 1172 may contain encoded information pertaining to the DNA strand 1152 to which labels 1172 are attached. These labels 1172 and the encoded data are illustrated as multiple, differently-shaded circles. The labels 1172 may be light-emitting labels and attached to DNA strands 1152 according to well-known methods for attaching light-emitting labels to DNA strands. Alternatively, heavy-atom labels with binary- or bar-codes may be employed. These heavy-atom labels would differ from the heavy-atom tags in that they would comprise much larger balls of many high-atomic number atoms which could be imaged with much larger pixels than would be necessary for DNA sequencing where single heavy atoms must be imaged. Embodiments of the functionalized specimen support 1100 configured according to FIG. 11C may be used, for example, as specimen supports in method 600 described above.

FIG. 11D is a close-up of rectangular grid opening X in an embodiment of specimen support 1100 comprising pre-defined locations 1133 and labeled DNA molecules 1163. Attachment sites (not shown) within or proximate to each of pre-defined locations 1133 are functionalized to attach the DNA strands 1153 to support film 1143 at different pre-defined locations 1133. Labels 1173 of labeled DNA molecules 1163 may be the same as labeled molecules 1163, except that labeled molecules 1163 are affixed to support film 1143 at pre-defined locations 1133. Embodiments of the functionalized specimen support 1100 configured according to FIG. 11D may be used, for example, as specimen supports in method 400 described above. Methods 200 and 400 may advantageously use the restriction of the locations of DNA strands to specific parts of the support surface provided by the specimen supports of FIGS. 11B and 11D, respectively to locate the DNA strands faster.

The DNA strands in FIGS. 11B, 11C, and 11D may generally be oriented in a single direction. For example, the DNA strands in FIGS. 11B, 11C, and 11D may generally be oriented in the direction of arrows 1181, 1182, and 1183, respectively.

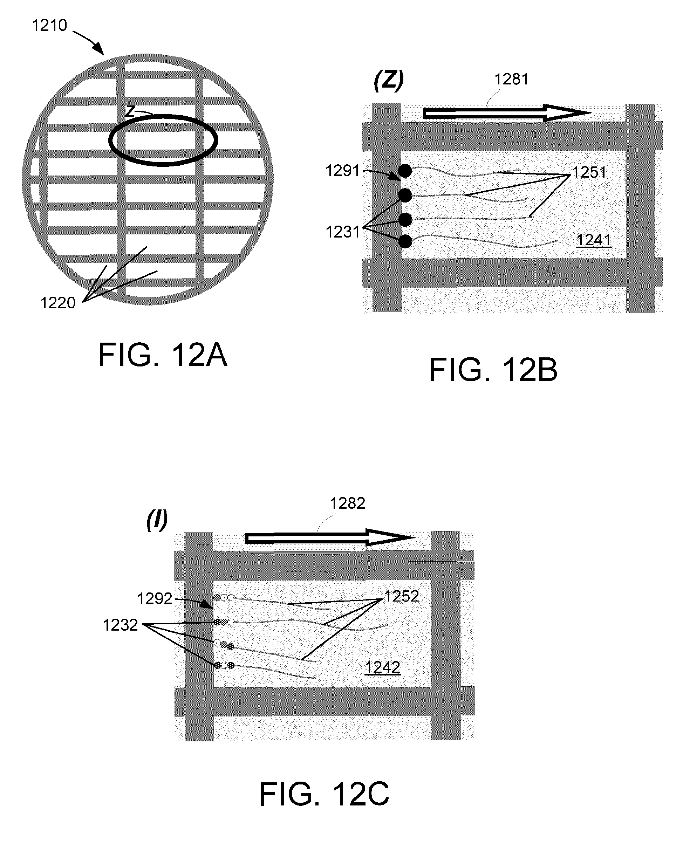

FIG. 12A is a schematic illustration of a microscope grid 1210, which may be a 3 mm microscopic grid, with custom rectangular openings 1220, where the process for laying down generally straight DNA strands is oriented along the long axes of the rectangular openings. The support film is as described in FIGS. 11 and 12. Embodiments of microscopic grid 1210 may have benefits that are the same as described for FIG. 12A. The difference between the embodiments illustrated in FIGS. 11 and 12 is that predefined locations comprising attachment sites are located at edges of grid openings in FIG. 12, and are not patterned into the thin film substrate as in FIG. 11. The dimensions of the rectangular grids may be in a range of from about 25 $\mu$m to about 100 $\mu$m.

FIG. 12B is a close-up of a single rectangular grid opening Z with pre-defined locations 1231 comprising attachment sites at the right edges 1291 of grid lines, the pre-defined locations being indicated schematically by circles. DNA strands 1251 are shown, each DNA strand attached to the microscope grid 1210 at one of the pre-defined locations 1231 via action of the attachment sites contained therein. These attachment sites may be defined by various well-known nano-patterning processes which are not part of the present invention. The DNA strands are supported from below by film 1241. Embodiments of the microscope grid 1210 configured according to FIG. 12B may be used, for example, as specimens supports in method 200 described above.

FIG. 12C is a close-up of a single rectangular grid opening Z with pre-defined locations 1232 comprising attachment sites at the right edges 1292 of grid lines. Each of labeled DNA molecules 1252 is attached to one of the pre-defined locations via action of the attachment sites contained therein. The labels may be the same as in FIGS. 10D and 11D. The DNA strands are supported from below by film 1242. Embodiments of the microscope grid 1210 configured according to FIG. 12C may be used, for example, as specimen supports in method 400 described above.

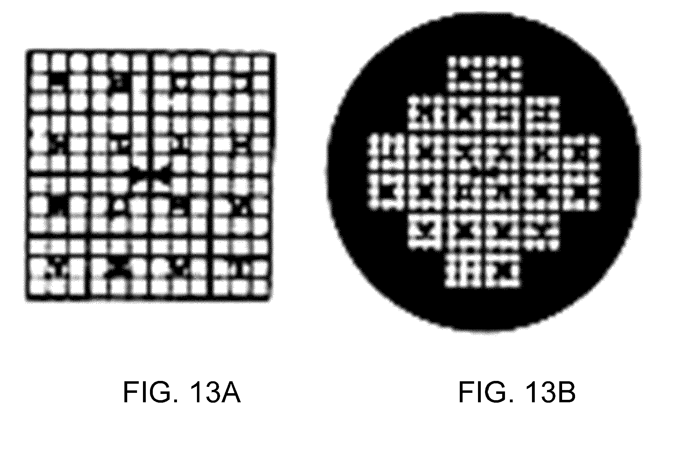

FIGS. 13A and 13B illustrate two exemplary electron microscope grids, taken from the Ted Pella, Inc., catalog, that may be adapted in accordance with embodiments of the disclosure. FIG. 13A is a "Finder" grid with letters etched into the grid mesh - these letters are large enough to see in a light microscope, but are not too large for the typical fields-of-view in an electron microscope. Finder grid 13B may be a 3 mm diameter finder grid. Thus these letters facilitate linking the sample preparation step (typically done using light microscopes) with the STEM locating and sequencing steps.

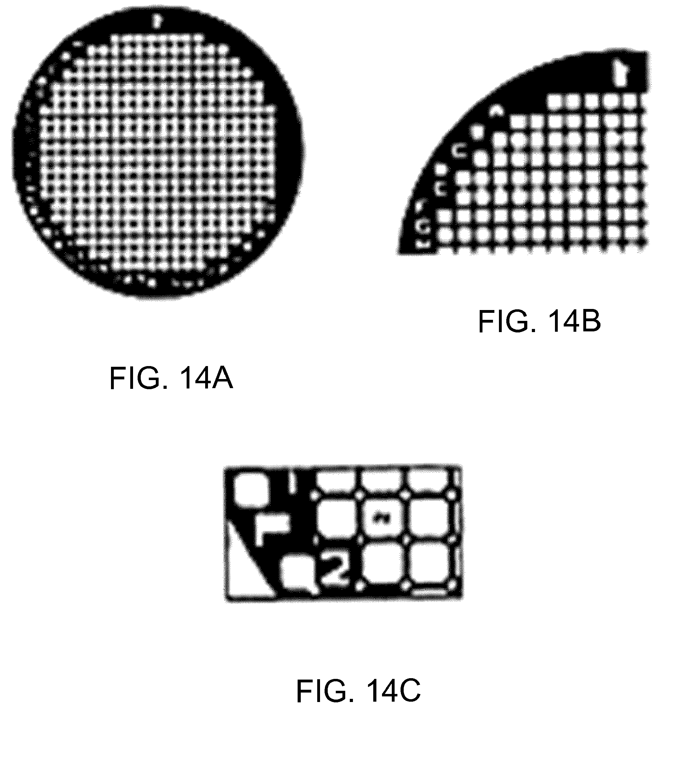

FIGS. 14A-14C show a 3 mm diameter Gilder Reference Locater Grid that may be adapted in accordance with embodiments of the disclosure. Each grid square has a unique alpha-numeric code to aid in locating samples having a pre-defined location on the grid. (Figure is from the Ted Pella, Inc. catalog).

Also disclosed herein is a system for locating and imaging specimens on a specimens support. The system may comprise one or more charged particle microscopes. Suitable microscope equipment includes, but is not limited to, a SEM, a TEM, a STEM, a light-optical microscope, or any combination thereof. The system may comprise one or more processors; and a computer-readable storage medium coupled to at least one of the one or more processors. The computer-readable storage medium includes first executable instructions that, when executed, cause the components of the system to direct one or more of methods 100-600 described above. The system may comprise one or more functionalized specimen supports described herein and/or one or more of the specimen preparations described herein.

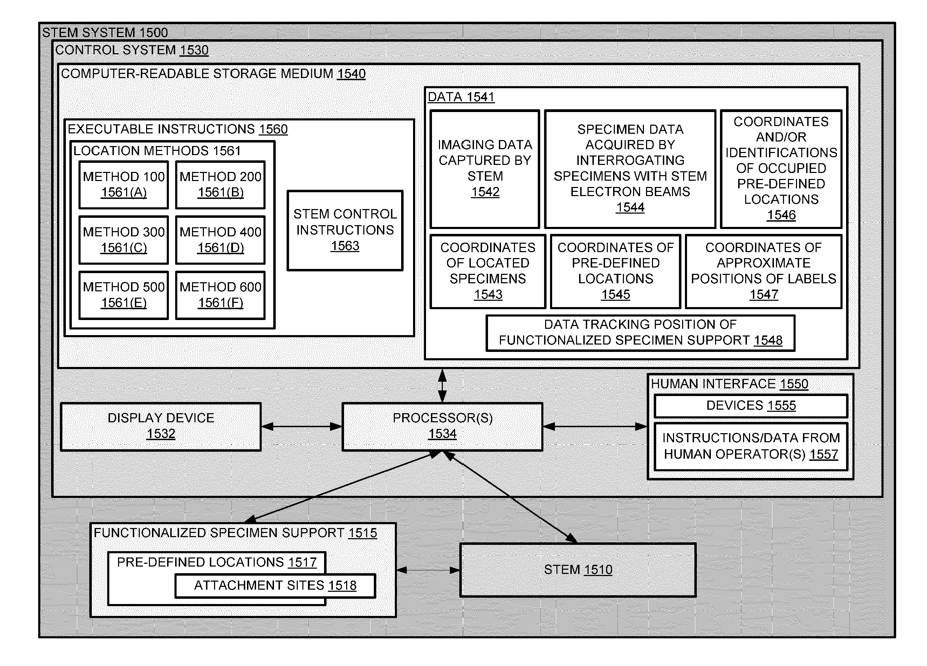

Turning to FIG. 15, a STEM system 1500 for sample processing according to an embodiment is shown. System 1500 comprises a STEM 1510, a functionalized specimen support 1515, and a control system 1530. Functionalized specimen support 1515 may comprise any of the functionalized specimen supports disclosed above.

Control system 1530 comprises a computer-readable storage medium 1540, a display device 1532, human interface 1550, and one or more processors 1534. Computer-readable storage medium 1540 stores processor accessible information comprising executable instructions 1560 and data 1541. Data 1541 includes imaging data captured by STEM 1510, coordinates of located specimens 1543, and specimen data 1544 acquired by interrogating specimens with electron beams of STEM 1510. Imaging data 1541 may also include coordinates of pre-defined locations 1545, coordinates and/or identifications 1546 of occupied pre-defined locations, coordinates of approximate positions of labels 1547, data tracking the position 1548 of functionalized specimen support 1515, or any combination thereof.

Computer-readable storage medium 1540 is coupled to one or more processors 1534 such that one or more processors 1534 can read and execute executable instructions 1560; process data acquired from at least one of STEM 1510, functionalized specimen support 1515 and human interface 1550 in accordance with executable instructions 1560; and direct the writing of data acquired from at least one of STEM 1510, functionalized specimen support 1515, and human interface 1550 to computer-readable storage medium 1540. Computer-readable storage medium 1540 may be a device capable of storing information for a period of time in a format readable by a computer, such as a hard drive. Examples of suitable computer-readable storage mediums include, but are not limited to, PATA, SATA, SCSI, and SSD hard drives.

Display device 1532 may be configured to display information from one or more of STEM 1510, functionalized specimen support 1515, and human interface 1550. Display device 1532 projects electronically generated images, which may include, for example, images of pre-defined locations, images of attachment sites, images of specimens located on functionalized specimen supports 1515, overlays calling out the presence of occupied and/or un-occupied pre-defined locations on one or more functionalized specimen supports, and combinations thereof. Examples of suitable display devices include, but are not limited to, LCD displays, plasma displays, cathode rays tube displays, and wall projectors.

Human interface 1550 comprises devices 1555 configured to enable human interaction with one or more of STEM 1510, functionalized specimen support 1515. Human interaction with STEM 1510, and functionalized specimen support 1515, for example, the inputting by a human operator of instructions and/or data 1557 for controlling STEM system 1500. Examples of devices suitable for human interface 1550 include, but are not limited to, keyboards, computer mice, touch interfaces such as tablet computers, or any combination thereof. Human interface 1550 also comprises executable instructions for managing the interactions of human operators with system 1500.

One or more processors 1534 carry out executable instructions 1560 stored on computer-readable storage medium 1540, thereby controlling STEM 1510 and functionalized specimen support 1515. One or more processors 1534 also read and send information to display device 1532 for viewing by a human operator and/or observer. One or more processors 1534 further process human-inputted instructions and/or data 1557 in accordance with executable instructions 1560 and direct the storage of data acquired from STEM 1510, and functionalized specimen supports 1515, and human interface 1550 on computer-readable storage medium 1540.

Embodiments of the method for locating specimens may be carried out by system 1500 according to executable instructions 1560 stored on computer-readable storage medium 1540 of system 1500. Executable instructions 1560 may comprise method instructions 1561(a)-1561(g) for carrying out one or more of methods 100, 200, 300, 400, 500, and 600, respectively. Executable instructions 1560 may include STEM control instructions 1563 for carrying out some or all of the STEM functionality described above.

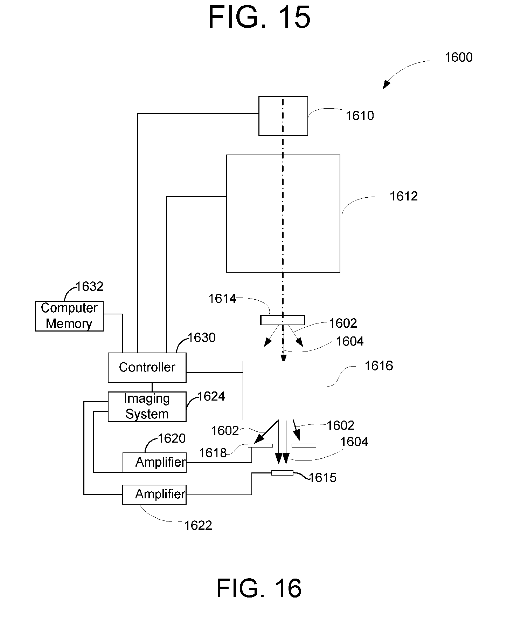

Turning to FIG. 16, a scanning transmission electron microscope (STEM) suitable for carrying out the methods of the disclosure are shown. FIG. 16 shows a STEM 1600 that can simultaneously detect dark-field electrons 1602 and bright-field electrons 1604. Dark-field electrons are electrons from the primary beam which have elastically scattered at least once off nuclei in the sample and thus have larger angles off-axis. Bright-field electrons are electrons from the primary beam which have either not scattered at all, or have only inelastically scattered off electrons in the sample, and thus have small angles off-axis. Microscope 1600 includes an electron source 1610 and a focusing column 1612 that focuses electrons from source 1610 into a small spot and scans the spot across a thin sample 1614 (i.e., a sample which is not thick enough to impede transmission of most of the electrons in the primary beam). The beam is composed of high energy electrons, that is, electrons having typical energies of between about 50 keV and 1,000 keV. Electrons that pass through sample 1614 enter projector 1616, which in some embodiments is not a separate lens, but is an extension below the sample 1614 of the lens field from a lens in focusing column 1612. For STEM applications, projector 1616 is typically adjusted so that bright-field electrons 1604, which passed through the sample with minimal deflection, impinge on disk-shaped bright field detector 1615, while dark-field electrons 1602, which were more strongly deflected by the sample, are detected by an annular dark-field STEM detector 1618. A signal from annular dark-field STEM detector 1618 is amplified by an amplifier 1620, and a signal from bright field detector 1615 is amplified by amplifier 1622. Signals from both amplifiers are sent to imaging system 1624, which can form an image of sample 1614 from the detected electrons. Both annular STEM detector 1618 and bright field detector 1615 can be a scintillator-photomultiplier detector or a solid state PIN detector. A controller 1630 can control the operation of microscope 1600, either manually in response to operator instructions or automatically in accordance with computer instructions stored in computer memory 1632. The computer instructions executed can perform any of the methods described herein.

Also disclosed herein, is a computer program product comprising instructions for executing one or more of the methods disclosed above (e.g., methods 100-600). The flowcharts and block diagrams in the different depicted embodiments illustrate the architecture, functionality, and operation of some possible implementations of apparatuses, methods and computer program products. In this regard, each block in the flowchart or block diagrams may represent a module, segment, or portion of computer usable or readable program code of a computer program product, which comprises one or more executable instructions for implementing the specified function or functions.

The computer program product may comprise one or more computer readable storage media having computer usable program code embodied therein to implement the functionality disclosed above. The computer program product may comprise data structures, executable instructions, and other computer usable program code. The computer program product may be embodied in a non-transitory computer readable medium. The computer program product may be embodied in removable computer storage media and/or non-removable computer storage media. The removable computer readable storage medium may comprise, without limitation, a paper tape, a magnetic tape, magnetic disk, an optical disk, a solid state memory chip, for example analog magnetic tape, compact disk read only memory (CD-ROM) disks, floppy disks, jump drives, digital cards, multimedia cards, and others.

The computer program product may be suitable for loading onto a computer system comprising control system 1530. Once loaded, the one or more processors 1534 may process the executable instructions and/or data structures in part by directly accessing the computer program product, for example by reading from a CD-ROM disk inserted into a disk drive peripheral of the computer system 1534. Alternatively, the one or more processors 1534 may process the executable instructions and/or data structures by remotely accessing the computer program product, for example by downloading the executable instructions and/or data structures from a remote server through the network connectivity devices. The computer program product may comprise instructions that promote the loading and/or copying of data, data structures, files, and/or executable instructions from the computer program product to, for example, computer-readable storage medium 1540.

The following are nonlimiting, specific embodiments in accordance with the present disclosure:

A first embodiment, which is a functionalized specimen support for use in charged particle microscopy, the functionalized specimen support comprising a specimen support surface configured to support a specimen during an interrogation of the specimen with a charged particle microscope, the specimen support surface comprising a functionalized site configured to maintain a position of the specimen at the functionalized site by way of attachment, attraction, or a combination thereof.

A second embodiment, which is the functionalized specimen support of the first embodiment, wherein the functionalized site is a first functionalized site and further comprising:

a plurality of functionalized sites comprising the first functionalized site, each of the functionalized sites being located at a different position on the specimen support surface and configured to maintain a position of a specimen at the functionalized site by way of attachment, attraction, or a combination thereof.

A third embodiment, which is the functionalized specimen support of the second embodiment, further comprising a second functionalized site of the plurality of functionalized sites, wherein the first functionalized site is configured to maintain a position of a specimen having a first property, the second functionalized site is configured to maintain a specimen having a second property, and the first property is different from the second property.

A fourth embodiment, which is the functionalized specimen support of the third embodiment, wherein the functionalized specimen support comprises a grid structure and a carbon film, wherein the carbon film comprises the specimen support surface and is supported by the grid.

A fifth embodiment, which is the functionalized specimen support of the third embodiment, wherein the functionalized specimen support comprises a grid structure and the specimen support surface is a surface of the grid structure.

A sixth embodiment, which is the functionalized specimen support of the first embodiment, wherein the functionalized site comprises a chemical functional group configured to react with the specimen and form a chemical bond between the functionalized site and the specimen such that the specimen is attached to the functionalized site.

A seventh embodiment, which is the functionalized specimen support of the first embodiment, wherein the functionalized site comprises a nanoscale interlocking mechanism configured to fixedly engage a feature of the specimen such that the specimen is attached to the functionalized site.

An eighth embodiment, which is the functionalized specimen support of the first embodiment, wherein the functionalized site is configured to attract and adhere the specimen to the functionalized site by an adhesive force, an intermolecular force, an electrostatic force, or any combination thereof.

A ninth embodiment, which is a specimen preparation for an electron microscope, comprising:

a specimen support, wherein a surface of the specimen support comprises a plurality of sites, each site being functionalized to maintain a position of a specimen at the site by way of a localized attractive force, an attachment mechanism, or a combination thereof; and
a plurality of specimens, each specimen adhered to one of the sites.

A tenth embodiment, which is the specimen preparation of the ninth embodiment, further comprising a plurality of labels, wherein each label is associated with one of the specimens and detectable with a microscope at a resolution lower than a minimum resolution necessary to determine the position of the specimen in an absence of the label.

An eleventh embodiment, which is the specimen preparation of the tenth embodiment, wherein each specimen comprises a DNA strand and the plurality of labels comprises light-emitting labels.

A twelfth embodiment, which is the specimen preparation of the eleventh embodiment, wherein the microscope is a light microscope.

A thirteenth embodiment, which is the specimen preparation of the tenth embodiment, wherein each label comprises information uniquely identifying the specimen associated with the label and the information can be extracted from the label by a microscope at resolutions lower than a minimum resolution necessary to identify the specimen in an absence of the label.

A fourteenth embodiment, which is the specimen preparation of the thirteenth embodiment, wherein the microscope is a scanning transmission electron microscope.

A fifteenth embodiment, which is the specimen preparation of the fourteenth embodiment, wherein for each specimen:

the specimen comprises a DNA strand; and
the label associated with the specimen comprises one or more heavy-atom tags, each of the one or more heavy-atom tags being located at a position of a base of the DNA strand and configured to produce a signal in response to an interrogation of the DNA strand by the scanning transmission electron microscope, the signal having an intensity indicative of the species of the base.

A sixteenth embodiment, which is the specimen preparation of the ninth embodiment, further comprising a readable non-transitory storage medium separate from the specimen support and comprising coordinates for each of the sites.

A seventeenth embodiment, which is the specimen preparation of the tenth embodiment, wherein the specimen support comprises a finder grid and the plurality of labels comprise letters etched into a grid mesh of the finder grid and perceivable through a light microscope.

A eighteenth embodiment, which is the specimen preparation of the ninth embodiment, wherein no two specimens are attached to a same site.

A nineteenth embodiment, which is the specimen preparation of the ninth embodiment, wherein a total quantity of the sites is greater than a total quantity of the specimens.

A twentieth embodiment, which is a method of locating specimens on a specimen support, comprising:

locally scanning a plurality of pre-defined locations on a specimen support for a presence of a specimen using a charged particle beam, wherein a position of a portion of the specimen is fixed at one of the pre-defined locations.

A twenty-first embodiment, which is the method of the twentieth embodiment, wherein the specimen comprises a DNA strand and the charged particle beam is an electron beam of a STEM having a resolution greater than or equal to a minimum resolution required to determine a position of a portion of the DNA strand and less than a minimum resolution required to sequence the DNA strand.

A twenty-second embodiment, which is the method of the twentieth embodiment, wherein locally scanning a plurality of pre-defined locations on a specimen support for a presence of a specimen using a charged particle beam comprises locally imaging the pre-defined locations using a charged particle beam at a resolution greater than or equal to a minimum resolution required to determine a position of a portion of the specimen on the specimen support and less than a minimum resolution required to analyze the specimen.

A twenty-third embodiment, which is the method of the twenty-second embodiment, further comprising determining a position of a portion of the specimen by processing imaging information obtained by locally imaging the pre-defined locations.

A twenty-fourth embodiment, which is the method of the twentieth embodiment, wherein the specimen comprises a label and locally scanning a plurality of pre-defined locations on a specimen support for a presence of a specimen using a charged particle beam comprises:

locally scanning the plurality of pre-defined locations for a presence of the label using a charged particle beam at a resolution greater than or equal to a minimum resolution required to determine the presence of the label and less than a minimum resolution required to determine a position of a portion of the specimen on the specimen support.

A twenty-fifth embodiment, which is the method of the twenty-fourth embodiment, further comprising scanning a pre-defined location indicating the presence of the label using a charged particle beam at a resolution greater than or equal to a minimum resolution required to determine a position of a portion of the specimen and less than a minimum resolution required to analyze the specimen.

A twenty-sixth embodiment, which is the method of the twentieth embodiment, wherein:

the plurality of pre-defined locations comprises a first pre-defined location and a second pre-defined location; and the specimen is a first specimen and a position of a portion of the first specimen is fixed at the first pre-defined location, and further comprising:

locally scanning the plurality of pre-defined locations for a presence of a second specimen using the charged particle beam, wherein a position of a portion of the second specimen is fixed at one of the second pre-defined locations.

A twenty-seventh embodiment, which is the method of the twenty-sixth embodiment, wherein:

the first specimen comprises a first DNA strand and a first label; the second specimen comprises a second DNA strand and a second label; and a resolution of the charged particle beam is greater than or equal to a minimum resolution required to detect a presence of at least one of the first label and the second label and less than a minimum resolution required to determine a position on the surface of a portion of at least one of the first DNA strand and the second DNA strand.

A twenty-eighth embodiment, which is the method of the twenty-seventh embodiment, wherein:

the first label comprises first heavy-atom tags configured to encode information particular to the first DNA strand, the second label comprises heavy-atom tags configured to encode information particular to the second DNA strand, and the charged particle beam is an electron beam of a transmission scanning electron microscope.

A twenty-ninth embodiment, which is the method of the twentieth embodiment, further comprising:

generating an image of one of the predefined locations from information obtained by locally scanning the plurality of pre-defined locations; and evaluating the image for indications of a presence of the specimen.

A thirtieth embodiment, which is the method of the twentieth embodiment, wherein the plurality of pre-defined locations are on a surface of the specimen support comprising a plurality of attachment sites, each attachment site occupying one of the plurality of pre-defined locations and functionalized to fix the position of the portion of the specimen at the pre-defined location of the attachment site upon exposure of the specimen to the attachment site.

A thirty-first embodiment, which is the method of the twentieth embodiment, wherein the plurality of pre-defined locations are on a surface of the specimen support and wherein a total area scanned by the charged particle beam is substantially smaller than a total area of the surface of the specimen support.

A thirty-second embodiment, which is a method of locating specimens on a specimen support, comprising:

loading a microscope with a specimen support having a surface comprising a plurality of pre-defined attachment locations, wherein a specimen comprising a light-emitting label is attached to one of the pre-defined attachment locations;

capturing local images of areas of the surface, each local image encompassing one of the pre-defined attachment locations and captured at a resolution sufficient to detect light from the light-emitting label; and

determining which of the pre-defined attachment locations is occupied by the specimen by evaluating the local images for a presence of a light signature of the light-emitting label.

A thirty-third embodiment, which is the method of the thirty-second embodiment, wherein the resolution is a first resolution and the first resolution is greater than or equal to a minimum resolution required to detect light from the light-emitting label and less than a minimum resolution required to determine a position of the specimen, and further comprising:

capturing an image of an area surrounding the pre-defined attachment location having the specimen attached at a second resolution sufficient to determine the position of the specimen; and

processing the image at the second resolution to determine the position of the specimen.

A thirty-fourth embodiment, which is the method of the thirty-fourth embodiment, wherein the specimen comprises a DNA strand and the second resolution is greater than or equal to a minimum resolution required to determine a position of the DNA strand and less than a minimum resolution required to sequence the DNA strand, and further comprising:

interrogating the specimen with a charged particle beam at a third resolution sufficient to sequence the DNA strand.

A thirty-fifth embodiment, which is the method of the thirty-second embodiment, wherein capturing local images of areas of the surface comprises capturing the local images with an optical microscope.

A thirty-sixth embodiment, which is a method of locating specimens on a specimen support, comprising:

capturing an image of a surface of a specimen support populated with labeled specimens, wherein a resolution of the image is sufficient to determine an approximate location of a label of one of the labeled specimens from the image; and

processing the image to determine an approximate position of the label.

A thirty-seventh embodiment, which is the method of the thirty-sixth embodiment, wherein the resolution is a first resolution, and further comprising:

capturing a localized image of an area surrounding the label at a second resolution sufficient to determine a position of the labeled specimen from the localized image; and

processing the image to determine the position of the labeled specimen from the localized image,

wherein the first resolution is greater than or equal to a minimum resolution required to determine the approximate position of the label from the image and less than a minimum resolution required to determine the position of the specimen.

A thirty-eighth embodiment, which is the method of the thirty-seventh embodiment, wherein the labeled specimen comprises a DNA strand and the second resolution is greater than or equal to a minimum resolution required to determine a position of the DNA strand and less than a minimum resolution required to sequence the DNA strand, and further comprising:

interrogating the labeled specimen with an electron beam emitted from a scanning transmission electron microscope at a third resolution sufficient to sequence the DNA strand.

A thirty-ninth embodiment, which is the method of the thirty-sixth embodiment, wherein capturing an image of the surface comprises capturing the image of the surface using an optical microscope.

A fortieth embodiment, which is a non-transitory machine-readable storage medium comprising executable instructions that, when executed, cause one or more processors to:

direct a charged particle beam to locally scan a plurality of pre-defined locations on a surface of a specimen support for a presence of a specimen using a charged particle beam, wherein the specimen is attached to one of the pre-defined locations.

A forty-first embodiment, which is the non-transitory machine-readable storage medium of the fortieth embodiment, wherein the specimen comprises a DNA strand and the charged particle beam is an electron beam of a STEM having a resolution greater than or equal to a minimum resolution required to determine a position of a portion of the DNA strand and less than a minimum resolution required to sequence the DNA strand.

A forty-second embodiment, which is the non-transitory machine-readable storage medium of the fortieth embodiment, wherein the executable instructions, when executed, cause the one or more processors to:

direct a charged particle beam to locally image the plurality of pre-defined locations at a resolution greater than or equal to a minimum resolution required to determine a position of a portion of the specimen on the surface and less than a minimum resolution required to analyze the specimen.

A forty-third embodiment, which is the non-transitory machine-readable storage medium of the forty-second embodiment, wherein the executable instructions, when executed, cause the one or more processors to process images of the pre-defined locations obtained by the charged particle beam to determine a position of a portion of the specimen on the surface.

A forty-fourth embodiment, which is the non-transitory machine-readable storage medium of the fortieth embodiment, wherein the specimen comprises a label, and
wherein the executable instructions, when executed, cause the one or more processors to direct the charged particle beam to locally scan the plurality of pre-defined locations for the presence of the label at a resolution greater than or equal to a minimum resolution required to determine the presence of the label and less than a minimum resolution required to determine a position of a portion of the specimen on the surface.

A forty-fifth embodiment, which is the non-transitory machine-readable storage medium of the forty-fourth embodiment, wherein the executable instructions, when executed, cause the one or more processors to direct a charged particle beam to scan a pre-defined location indicating the presence of the label at a resolution greater than or equal to a minimum resolution required to determine a position of a portion of the specimen on the surface and less than a minimum resolution required to analyze the specimen.

A forty-sixth embodiment, which is the non-transitory machine-readable storage medium of the fortieth embodiment, wherein:

the plurality of pre-defined locations comprises a first pre-defined location and a second pre-defined location;
the specimen is a first specimen and a position of a portion of the first specimen is fixed at the first pre-defined location; and
the executable instructions, when executed, cause the one or more processors to:

direct a charged particle beam to locally scan the plurality of pre-defined locations for a presence of a second specimen, wherein a position of a portion of the second specimen is fixed at one of the second pre-defined locations.

A forty-seventh embodiment, which is the non-transitory machine-readable storage medium of the forty-sixth embodiment, wherein:

the first specimen comprises a first DNA strand and a first label;
the second specimen comprises a second DNA strand and a second label; and
a resolution of the charged particle beam is greater than or equal to a minimum resolution required to detect a presence of at least one of the first label and the second label and less than a minimum resolution required to determine a position on the surface of a portion of at least one of the first DNA strand and the second DNA strand.

A forty-eighth embodiment, which is the non-transitory machine-readable storage medium of the forty-seventh embodiment, wherein:

the first label comprises first heavy-atom tags configured to encode information particular to the first DNA strand, the second label comprises second heavy-atom tags configured to encode information particular to the second DNA strand, and
the charged particle beam is an electron beam of a transmission scanning electron microscope.

A forty-ninth embodiment, which is the non-transitory machine-readable storage medium of the fortieth embodiment, wherein the executable instructions, when executed, cause the one or more processors to:

generate an image of one of the predefined locations from information obtained by locally scanning the plurality of pre-defined locations; and
evaluate the image for indications of a presence of the specimen.

A fiftieth embodiment, which is a scanning transmission electron microscope system for high-throughput sample processing, comprising:

a scanning transmission electron microscope;
a specimen support, wherein a surface of the specimen support comprises a plurality of locations, each location comprising an attachment site functionalized to fix a position of a portion of a specimen to the surface at the location.

A fifty-first embodiment, which is the system of the fiftieth embodiment, wherein the plurality of locations are pre-defined.
A fifty-second embodiment, which is the system of the fifty-first embodiment, further comprising:

one or more processors; and
a computer-readable storage medium coupled to at least one of the one or more processors, the computer-readable storage medium comprising executable instructions that, when executed, cause the one or more processors to direct an electron beam of the scanning transmission electron microscope to locally scan the pre-defined locations to determine which of the pre-defined locations are occupied by a specimen, to determine a position of a specimen on the surface, or a combination thereof.

A fifty-third embodiment, which is the system of the fiftieth embodiment, wherein each attachment site is functionalized to fix an end of a strand of DNA to the surface.
While embodiments of the invention have been shown and described, modifications thereof can be made by one skilled in the art without departing from the teachings of the invention. The embodiments described herein are exemplary only, and are not intended to be limiting. Many variations and modifications of the invention disclosed herein are possible, and alternative embodiments that result from combining, integrating, and/or omitting features of the embodiments disclosed herein are also within the scope of the invention. Where numerical ranges or limitations are expressly stated, such express ranges or limitations should be understood to include iterative ranges or limitations of like magnitude falling within the expressly stated ranges or limitations (e.g., from about 1 to about 10 includes, 2, 3, 4, etc.; greater than 0.10 includes 0.11, 0.12, 0.13, etc.). For example, whenever a numerical range with a lower limit, Rl and an upper limit, Ru, is disclosed, any number falling within the range is specifically disclosed. In particular, the following numbers within the range are specifically disclosed: R=Rl+k*(Ru-Rl),wherein k is a variable ranging from 1 percent to 100 percent with a 1 percent increment, i.e., k is 1 percent, 2 percent, 3 percent, 4 percent, 5 percent, 50 percent, 51 percent, 52 percent, 95 percent, 96 percent, 97 percent, 98 percent, 99 percent, or 100 percent. Moreover, any numerical range defined by two R numbers as defined in the above is also specifically disclosed. Use of the term "optionally" with respect to any element of a claim is intended to mean that the subject element is required, or alternatively, is not required. Both alternatives are intended to be within the scope of the claim. Use of the term "may" to introduce features of embodiments of the disclosure (*e.g.,* "In an embodiment, the widget *may* be connected to a cog,") is intended to mean that embodiments reciting said features are considered to be within the scope of the invention and such embodiments shall be construed as being positively recited by the specification. However, use of the term "may" to introduce features of embodiments is not an indication that embodiments failing to recite said features are considered outside the scope of the invention. Further, although various features of embodiments are described in plural form (*e.g.,* attachment surfaces, localized attractive sites, etc.), embodiments having single instances of said features (*e.g.,* one attachment surface, one localized attractive site, etc.), alone or in combination with single or plural instances of other features, are also contemplated to be within the scope of the invention unless explicitly indicated otherwise. Use of broader terms such as "comprises," "includes," "having," etc. should be understood to provide support for narrower terms such as "consisting of," "consisting essentially of," "comprised substantially of," etc.

Although the present invention and its advantages have been described in detail, it should be understood that various changes, substitutions and alterations can be made to the embodiments described herein without departing from the scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

Accordingly, the protection is not limited by the description set out above but is only limited by the claims which follow, that scope including all equivalents of the subject matter of the claims. Each and every claim is incorporated into the specification as an embodiment of the present invention. Thus, the claims are a further description and are an addition to the embodiments of the present invention. The discussion of a reference in the Detailed Description of the Embodiments is not an admission that it is prior art to the present invention, especially any reference that may have a publication date after the priority date of this application.

**Claims**

1. A functionalized specimen support (1000) for use in charged particle microscopy, the functionalized specimen support comprising a specimen support surface configured to support a specimen during an interrogation of the specimen with a charged particle microscope (1600),
   **characterized in that:**

   the specimen support surface comprises a functionalized site configured to maintain a position of the specimen (1051) at the functionalized site by way of attachment, attraction, or a combination thereof.

2. The functionalized specimen support of claim 1, wherein the functionalized site is a first functionalized site and further comprising:

   a plurality of functionalized sites comprising the first functionalized site, each of the functionalized sites being located at a different position on the specimen support surface and configured to maintain a position of a specimen at the functionalized site by way of attachment, attraction, or a combination thereof.

3. The functionalized specimen support of claim 2, further comprising a second functionalized site of the plurality of functionalized sites, wherein the first functionalized site is configured to maintain a position of a specimen having a first property, the second functionalized site is configured to maintain a specimen having a second property, and the first property is different from the second property.

4. The functionalized specimen support of claim 3, wherein the functionalized specimen support comprises a grid structure and a carbon film, wherein the carbon film comprises the specimen support surface and is supported by the grid.

5. The functionalized specimen support of claim 3, wherein the functionalized specimen support comprises a grid structure (1010) and the specimen support surface is a surface of the grid structure.

6. The functionalized specimen support of claim 1, wherein the functionalized site comprises a chemical functional group configured to react with the specimen and form a chemical bond between the functionalized site and the specimen such that the specimen is attached to the functionalized site.

7. The functionalized specimen support of claim 1, wherein the functionalized site comprises a nanoscale interlocking mechanism configured to fixedly engage a feature of the specimen such that the specimen is attached to the functionalized site.

8. The functionalized specimen support of claim 1, wherein the functionalized site is configured to attract and adhere the specimen to the functionalized site by an adhesive force, an intermolecular force, an electrostatic force, or any combination thereof.

9.  A method of locating specimens on a specimen support, comprising:

    locally scanning a plurality of pre-defined locations (1031) on a specimen support (1000) for a presence of a specimen using a charged particle beam (140; 240) wherein a position of a portion of the specimen is fixed at one of the pre-defined locations.

10. The method of claim 9, wherein the specimen comprises a DNA strand (1051) and the charged particle beam is an electron beam of a scanning transmission electron microscope (STEM) (1600) having a resolution greater than or equal to a minimum resolution required to determine a position of a portion of the DNA strand and less than a minimum resolution required to sequence the DNA strand (240).

11. The method of claim 9, wherein locally scanning a plurality of pre-defined locations on a specimen support for a presence of a specimen using a charged particle beam comprises locally imaging the pre-defined locations using a charged particle beam at a resolution greater than or equal to a minimum resolution required to determine a position of a portion of the specimen on the specimen support and less than a minimum resolution required to analyze the specimen (140), and further comprising determining a position of a portion of the specimen by processing imaging information obtained by locally imaging the pre-defined locations (150).

12. The method of claim 9, wherein the specimen comprises a label and locally scanning a plurality of pre-defined locations on a specimen support for a presence of a specimen using a charged particle beam comprises:

    locally scanning the plurality of pre-defined locations for a presence of the label using a charged particle beam at a resolution greater than or equal to a minimum resolution required to determine the presence of the label and less than a minimum resolution required to determine a position of a portion of the specimen on the specimen support (340a).

13. The method of claim 9, further comprising:

    generating an image of one of the predefined locations from information obtained by locally scanning the plurality of pre-defined locations; and
    evaluating the image for indications of a presence of the specimen.

14. The method of claim 9, wherein the plurality of pre-defined locations are on a surface of the specimen support comprising a plurality of attachment sites (1031), each attachment site occupying one of the plurality of pre-defined locations and functionalized to fix the position of the portion of the specimen at the pre-defined location of the attachment site upon exposure of the specimen to the attachment site.

15. The method of claim 9, wherein the plurality of pre-defined locations are on a surface of the specimen support and wherein a total area scanned by the charged particle beam is substantially smaller than a total area of the surface of the specimen support.

100

Expose Specimen(s) to Surface of Specimen Support — 110

Fix Positions of Specimen(s) at
Pre-Defined Locations on Surface of Specimen Support — 120

Mount Specimen Support in Charged Particle Microscope — 130

Locally Scan Areas of Pre-Defined Locations at
Resolution(s) Sufficient to Determine Positions of Specimen(s) — 140

Process Scan Data to Determine Positions of Specimen(s) — 150

Scan Specimen(s) at Resolution Sufficient to Analyze Specimens — 160

Process Scan Data to Obtain Information About Specimen(s) — 170

FIG. 1

200

| Expose DNA Strand(s) to Surface of Specimen Support | 210 |

↓

| Fix Position(s) of DNA Strand(s) at Different<br>Pre-Defined Locations on Surface of Specimen Support | 220 |

↓

| Mount Specimen Support in STEM | 230 |

↓

| Locally Image Areas of Pre-Defined Locations with STEM Beam at<br>Resolution(s) Sufficient to Determine Position(s) of DNA Strand(s) | 240 |

↓

| Process Image Data to Determine Position(s) of DNA Strand(s) | 250 |

↓

| Scan DNA Strand(s) with STEM Beam at<br>Resolution(s) Sufficient to Sequence DNA Strand(s) | 260 |

↓

| Process Scan Data of DNA Strand(s) to Sequence DNA Strand(s) | 270 |

# FIG. 2

300

Label Specimen(s) — 305

↓

Expose Labelled Specimen(s) to
Surface of Specimen Support — 310

↓

Fix Positions of Labelled Specimen(s) at
Pre-Defined Locations on Surface of Specimen Support — 320

Labels Detectable by
Charged Particle Microscope

Labels Detectable
by Light Microscope

340a

Locally Scan Areas of the Pre-Defined
Locations with Charged Particle Beam at
Resolution(s) Sufficient to Detect
Presence of Label(s)

Capture Images of Pre-Defined Locations
with Resolution(s) Sufficient to Detect
Presence of Label(s) using light
microscope — 340b

↓

↓

350a

Process & Evaluate Scan Data of
Individual Areas for Indications of
Presence of Label(s)

Process & Evaluate Images for
Presence of Label Signatures — 350b

↓

Locally Scan Occupied Pre-Defined Location(s) with
Charged Particle Beam at Resolution(s) Sufficient to
Determine Position(s) of Labelled Specimen(s) — 360

↓

Process Scan Data to Determine
Position(s) of Labelled Specimen(s) — 370

↓

Scan Labelled Specimen(s) with
Charged Particle Beam at Resolution(s)
Sufficient to Analyze Specimen(s) — 380

↓

Process Scan Data to Obtain
Information About Labelled Specimen(s) — 390

FIG. 3

FIG. 4

500

505

| Label Specimen(s) |
| --- |

510

| Populate Surface of Specimen Support with Labeled Specimen(s) |
| --- |

530

| Capture Image of Surface of Specimen Support at Resolution(s) Sufficient to Detect and Determine Approximate Positions of Label(s) |
| --- |

540

| Process Image of Surface to Determine Approximate Position(s) of Label(s) |
| --- |

550

| Locally Image Areas of Surface at Approximate Positions of Label(s) using Charged Particle Beam having Resolution(s) Sufficient to Determine Positions of Specimen(s) |
| --- |

560

| Process Localized Image(s) to Determine Position(s) of Specimen(s) |
| --- |

570

| Scan Specimen(s) with Charged Particle Beam at Resolution(s) Sufficient to Analyze Specimen(s) |
| --- |

580

| Process Scan Data to Obtain Information About Specimen(s) |
| --- |

FIG. 5

<u>600</u>

| | 605 |
|---|---|
| **Encode DNA Strand(s) with Label(s)** | |

| | 610 |
|---|---|
| **Populate Surface of Specimen Support with Labeled DNA Strand(s)** | |

Heavy-Atom Tags as Labels                    Light-Emitting Labels

630a

**Capture Image of Surface of Specimen Support with STEM Beam at Resolution(s) Sufficient to Detect and Determine Positions of Heavy-Atom Label(s)**

630b

**Capture Image of Surface of Specimen Support with Light Microscope at Resolution(s) Sufficient to Detect and Determine Positions of Light-Emitting Label(s)**

640

**Process Image of Surface to Determine Approximate Position(s) of Label(s)**

650

**Capture Localized Image(s) of Areas Surrounding Label(s) with STEM Beam at Resolution(s) Sufficient to Determine Positions of DNA Strand(s)**

660

**Process Localized Image(s) to Determine Position(s) of DNA Strand(s)**

670

**Scan DNA Strand(s) with STEM Beam at Resolution(s) Sufficient to Sequence DNA Strand(s)**

680

**Process Scan Data to Sequence DNA Strand(s)**

# FIG. 6

FIG. 7

FIG. 8

FIG. 9

FIG. 10A

FIG. 10B

FIG. 10C

FIG. 10D

FIG. 11A

FIG. 11B

FIG. 11C

FIG. 11D

1210

1220

# FIG. 12A

(Z)

1281

1291

1251

1231

1241

# FIG. 12B

(I)

1282

1292

1252

1232

1242

# FIG. 12C

FIG. 13A

FIG. 13B

FIG. 14B

FIG. 14A

FIG. 14C

STEM SYSTEM 1500

CONTROL SYSTEM 1530

COMPUTER-READABLE STORAGE MEDIUM 1540

EXECUTABLE INSTRUCTIONS 1560

LOCATION METHODS 1561

| METHOD 100 1561(A) | METHOD 200 1561(B) |
| METHOD 300 1561(C) | METHOD 400 1561(D) |
| METHOD 500 1561(E) | METHOD 600 1561(F) |

STEM CONTROL INSTRUCTIONS 1563

DATA 1541

| IMAGING DATA CAPTURED BY STEM 1542 | SPECIMEN DATA ACQUIRED BY INTERROGATING SPECIMENS WITH STEM ELECTRON BEAMS 1544 | COORDINATES AND/OR IDENTIFICATIONS OF OCCUPIED PRE-DEFINED LOCATIONS 1546 |
| COORDINATES OF LOCATED SPECIMENS 1543 | COORDINATES OF PRE-DEFINED LOCATIONS 1545 | COORDINATES OF APPROXIMATE POSITIONS OF LABELS 1547 |

DATA TRACKING POSITION OF FUNCTIONALIZED SPECIMEN SUPPORT 1548

HUMAN INTERFACE 1550

DEVICES 1555

INSTRUCTIONS/DATA FROM HUMAN OPERATOR(S) 1557

DISPLAY DEVICE 1532

PROCESSOR(S) 1534

FUNCTIONALIZED SPECIMEN SUPPORT 1515

PRE-DEFINED LOCATIONS 1517

ATTACHMENT SITES 1518

STEM 1510

FIG. 15

FIG. 16